(19) Europäisches Patentamt
European Patent Office
Office européen des brevets

(11) **EP 3 039 597 B1**

(12) **EUROPEAN PATENT SPECIFICATION**

(45) Date of publication and mention
of the grant of the patent:
**25.07.2018 Bulletin 2018/30**

(51) Int Cl.:
***G06F 19/18*** *(2011.01)*        ***G06F 19/26*** *(2011.01)*
***G06F 19/28*** *(2011.01)*

(21) Application number: **14761819.3**

(22) Date of filing: **28.08.2014**

(86) International application number:
**PCT/EP2014/068315**

(87) International publication number:
**WO 2015/028576 (05.03.2015 Gazette 2015/09)**

(54) **HAPLOTYPING AND COPY NUMBER TYPING USING POLYMORPHIC VARIANT ALLELIC FREQUENCIES**

HAPLOTYPISIERUNG UND KOPIENZAHLTYPISIERUNG MIT ALLELFREQUENZEN MIT POLYMORPHEN VARIANTEN

HAPLOTYPAGE ET TYPAGE DU NOMBRE DE COPIES À L'AIDE DE FRÉQUENCES D'ALLÈLES DE VARIANT POLYMORPHIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2013 US 201361871228 P**
**30.09.2013 US 201361960922 P**
**17.10.2013 GB 201318369**
**12.12.2013 US 201361915406 P**

(43) Date of publication of application:
**06.07.2016 Bulletin 2016/27**

(73) Proprietor: **Katholieke Universiteit Leuven**
**3000 Leuven (BE)**

(72) Inventors:
• **ZAMANI ESTEKI, Masoud**
**B-3001 Heverlee (BE)**
• **VERMEESCH, Joris**
**B-3020 Veltem-Beisem (BE)**
• **VOET, Thierry**
**B-3001 Heverlee (BE)**

(74) Representative: **Gevers Patents**
**Intellectual Property House**
**Holidaystraat 5**
**1831 Diegem (BE)**

(56) References cited:
**WO-A1-2011/157846     US-A1- 2008 318 235**
**US-A1- 2011 086 769     US-A1- 2011 105 353**
**US-A1- 2011 201 507     US-A1- 2011 288 780**
**US-A1- 2012 196 754**

• T. VOET ET AL: "Single-cell paired-end genome sequencing reveals structural variation per cell cycle", NUCLEIC ACIDS RESEARCH, vol. 41, no. 12, 29 April 2013 (2013-04-29), pages 6119-6138, XP055096338, ISSN: 0305-1048, DOI: 10.1093/nar/gkt345
• LI ET AL: "Association Mapping via Regularized Regression Analysis of Single-Nucleotide-Polymorphism Haplotypes in Variable-Sized Sliding Windows", THE AMERICAN JOURNAL OF HUMAN GENETICS, vol. 80, no. 4, 1 April 2007 (2007-04-01), pages 705-715, XP055151252, ISSN: 0002-9297, DOI: 10.1086/513205
• A. H. HANDYSIDE ET AL: "Karyomapping: a universal method for genome wide analysis of genetic disease based on mapping crossovers between parental haplotypes", JOURNAL OF MEDICAL GENETICS, vol. 47, no. 10, 25 October 2009 (2009-10-25), pages 651-658, XP055146321, ISSN: 0022-2593, DOI: 10.1136/jmg.2009.069971 cited in the application
• JIQIU CHENG ET AL: "Single-cell copy number variation detection", GENOME BIOLOGY, BIOMED CENTRAL LTD., LONDON, GB, vol. 12, no. 8, 19 August 2011 (2011-08-19), page R80, XP021109828, ISSN: 1465-6906, DOI: 10.1186/GB-2011-12-8-R80

EP 3 039 597 B1

• NIELS VAN DER AA ET AL: "Preimplantation genetic diagnosis guided by single-cell genomics", GENOME MEDICINE, vol. 5, no. 8, 19 August 2013 (2013-08-19) , pages 71-81, XP055150892,

## Description

### Field of the invention

The present invention relates to a method for haplotyping and/or copy number typing of genetic material. More specifically, the present invention relates to a method for genome-wide haplotyping, haplotype-specific DNA-copy number profiling, and determining the meiotic/mitotic origins of DNA-anomalies by determining, phasing and segmenting polymorphic variant (PV) allele fractions (PVAF) in single cells, pools of few cells, multi-cell DNA preparations, or even DNA preparations from cell-free DNA in e.g. the blood stream.

### Background of the invention

The development of (single-cell) diagnostic tests targeted at a particular mutation for each family specifically is time-consuming, labor intensive and costly, leading in addition to long waiting lists for the couples to undergo the procedure. Hence, novel generic methods for genetic diagnosis are imperative.

Chromosome aneuploidy is a major cause of pregnancy loss and abnormal development of a fetus or individual. Such aneuploidies may result from meiotic errors, which are more prevalent in oogenesis in the decade preceding the menopause. Preimplantation genetic screening (PGS) has been conceptualized to increase the pregnancy rates per embryo transferred and to prevent abnormal pregnancies and diseased live births following IVF. However, FISH- and aCGH-based PGS methods do not allow discriminating between meiotic or mitotic errors. In particular, the cleavage stage cell divisions are prone to mitotic chromosome segregation errors, which not necessarily impairs normal embryonic development.

Genome-wide SNP-typing, based on microarrays or next-generation sequencing, and subsequent genome-wide haplotyping, has recently become feasible. However, precise haplotyping of the entire genome of a non-metaphase diploid single cell has been largely precluded, mainly due to genotype errors introduced by single-cell DNA-amplification artifacts like random ADO and preferential amplification (PA) of one allele over the other, as well as by false algorithmic interpretations of SNP-probe intensities.

Wang et al. in "PennCNV: an integrated hidden Markov model designed for high-resolution copy number variation detection in whole-genome SNP genotyping data." Genome research 17, 1665 (2007), developed an integrated hidden Markov model, called PennCNV, for determining copy-number aberrations. PennCNV uses SNP B-allele frequency (BAF)- and logR-values to determine copy-number aberrations. This works well for multi-cell samples which have not been subjected to whole genome amplification (WGA), as there are specific patterns of BAF-values for different copy number aberrations. However, PVAF-values obtained from a whole-genome amplification (WGA) of a single cell can be significantly distorted due to allelic amplification bias, and hence are not as distinctive for different copy-number states as PVAF values derived from a non-WGAed DNA sample. In particular duplications and trisomies are extremely difficult to affirm from ordinary PVAF and logR-values in single cells; which might lead to misinterpretations in single-cell copy number analysis, and hence even to misdiagnoses when applied in a clinical setting. PennCNV and other similar approaches known in the art, can also employ trio genotypes to determine the origin of aberrations in DNA-samples derived from a large amount of cells. Although these approaches work well for detecting and affirming deletions, using discrete bi-allelic genotypes of a multi-cell DNA sample, neither inference of parental-origin nor of mechanistic-origin of the aberrations, in particular of duplications, can be accurately determined. In embodiments of the present invention, however, the informative PVAF-values are advantageously haplotyped / phased and utilized for the determination of haplotype-specific copy number states as well as their parental- and mechanistic-origin, down to single-cell level.

In addition to these methods known in the art, there are also population-based methods known in the art for the determination of allelic imbalances and mosaicisms in multi-cell DNA samples not subjected to WGA. Vattathil et al. in "Haplotype-based profiling of subtle allelic imbalance with SNP arrays", Genome research 23, 152, (2013), use B-allele frequency (BAF)-values of heterozygous SNP-calls to detect alteration from the normal one-to-one allele ratio. Following population-based statistical estimation of the germline haplotypes using fast-PHASE as described by Scheet et al in "A fast and flexible statistical model for large-scale population genotype data: applications to inferring missing genotypes and haplotypic phase." in American journal of human genetics 78, 629 (2006), they compared these estimated germline haplotypes and the "excess" haplotypes deduced from BAF-values of the same heterozygous SNPs surpassing a threshold (i.e. the median of observed BAFs at all heterozygous loci) to determine phase concordance between the germline haplotype and the BAF-deduced "excess" haplotype, and thus the haplotype-specific allelic imbalances. However, they do not consider the magnitude of the BAFs per se and ignore the total intensities. In contrast, Nik-Zainal et al. in "The life history of 21 breast cancers." Cell, 149, 5, 2012, elegantly applied the data from the 1000 genome project ("A map of human genome variation from population-scale sequencing." Nature 467, 1061, 2010) and used IMPUTE (Howie et al. in "Fast and accurate genotype imputation in genome-wide association studies through pre-phasing." Nature Genetics 44, 8, 2012) to phase germline SNPs determined from next-generation sequencing data into fragmented and thus short parent-specific haplotype blocks. Subsequent analysis of allelic ratios by haplotype demonstrated higher sensitivity to detect allelic imbalances and anomalies than when individual SNP BAFs would be scored genome wide. By applying this principle (named as the Battenberg algorithm) on BAF-values of heterozygous SNP-calls, they could evaluate the distortion of BAF-values of heterozygous SNP-calls from the expected 0.5 value to determine allelic imbalances in short haplotype stretches, and hence investigate mosaic DNA-copy number aberrations in a DNA-sample. Importantly however, the above two methods known in the art have been shown to work on standard DNA-samples extracted from a large amount of cells, but will be inefficient on BAF-values derived from single-cell data as the population-determined germline haplotypes represent short stretches, and the required whole-genome amplification processes for single-cell analysis introduce noise in the SNP's BAF. In addition, parental homologous recombination sites cannot be revealed in the single-cell sample. In contrast, embodiments of the present invention advantageously apply family or relative-based phasing principles and in particular does not need determination of the SNP-calls of the DNA-sample under study per se. Hence, the method according to embodiments of the invention advantageously can interpret PVAF-values over extensive germline haplotypes, and can be used to interpret PVAF-values of a single cell, a few cells or DNA-samples requiring whole-genome amplifications, in addition to standard DNA-samples. The method according to embodiments of the invention advantageously applies the robust discrete PV genotypes of the parents, and in specific embodiments of an additional close relative to phase the parental genotypes, which are subsequently applied to study the PVAF-values of a DNA sample from paternal and maternal informative SNPs, respectively, in a haplotype specific way. The method according to embodiments of the invention uses long parental haplotype blocks where the influence of the stochastic WGA-artifacts can be accounted for. In addition, these population-based methods cannot effectively trace the mechanistic origin of genomic anomalies. However, the present invention advantageously can trace the genomic anomalies to meiosis or mitosis errors. This has important implications in preimplantation genetic diagnosis (PGD), in particular when the diagnosis is done at the cleavage stage in early development. If a single cell of a preimplantation embryo has an aberration with a meiotic mechanistic origin, that embryo should not be transferred, as the aberration is most likely perpetuated in all single blastomeres of the embryo. However, if a cleavage-stage embryo has an aberration with mitotic mechanistic origin, this would not necessarily mean that the aberration is present in all single blastomeres of that embryo, as chromosomal instability is common in cleavage-stage embryogenesis. Thus, determining meiotic or mitotic mechanistic origin of chromosomal anomalies by the present invention enables preimplantation genetic diagnosis for aneuploidy screening (PGS) for cleavage-stage embryos. The present invention, therefore, makes simultaneous PGD and PGS in one assay feasible.

Navin et al. in "Tumor evolution inferred by single-cell sequencing" Nature 472, 90 (2011), developed a focal sequence read depth analysis method to compute single-cell DNA copy number landscapes following sequencing of a single-cell WGA product. The amount of single-end sequence reads corresponding to specific bins were computed to determine logR-values and subsequent copy-number states of WGAed single-cell genomes. Advantageously embodiments of the present invention uses PV genotypes, logR- and PVAF-values derived from high throughput genotyping technologies, including SNP-arrays and next generation sequencing devices, and integrated logR- and PVAF-values to show genomic aberrations. The latter embodiment works well for the determination of a deletion, however ordinary single-cell PVAF-values are not robust for affirmation of duplications. In comparison to the latter embodiment, other embodiments of the invention categorize and subcategorize the PVAF-values followed by segmenting of these values. Thus, embodiments of the method of the invention advantageously can reconstruct the parental haplotypes and determines the meiotic or mitotic mechanistic origin of aberrations.

For haplotyping of single cells or low cell amounts following WGA, prior art methods can make use of discrete bi-allelic genotypes of the cell. In these approaches, the determination of accurate allelic and haplotype quantities and their origin are not possible. For instance, these methods known in the art cannot accurately distinguish a diploid chromosome from a trisomy with mitotic origin. Furthermore, when admixtures of cells are present in a few-cell or multi-cell DNA-sample, the mosaic nature of the DNA-sample cannot be dissected. Furthermore, since these approaches utilize bi-allelic genotypes, they can severely suffer from WGA artifacts as well as genuine DNA copy number variants in the sample. To alleviate WGA allele drop out artifacts to some extent, Handyside et al. in "Karyomapping: a universal method for genome wide analysis of genetic disease based on mapping crossovers between parental haplotypes." J Med Genet, 47, 10, (2010), suggested the use of only heterozygous genotypes. Although this approach can vanish the influence of ADO artifacts; it does not alleviate false heterozygous genotypes that are generated by allele drop in (ADI) artifacts. Since, heterozygous SNPs in a WGA product only make up a small proportion of a (single-cell) genotype, minor ADI-artifacts might have a large effect leading to false haplotypes. Voet et al. in "Single-cell paired-end genome sequencing reveals structural variation per cell cycle." Nucleic Acids Res, 41, 12, (2013), discloses methods of analyzing the genetic material of neurons to identify aneuploid neurons as well as sub-chromosomal copy number variations, comprising obtaining sequence data by Whole Genome Amplification of single cells and computing the B-allele fraction (BAF) of SNPs as the number of reads incorporating the SNP B allele divided by the number of reads spanning the SNP. BAF values are an embodiment of PVAF values. The authors were able to detect the DNA imbalances resulting from an unbalanced inheritance of the paternal derivative chromosome 16 and the matching cluster of discordantly mapping read pairs. Copy number profiling of single cells was carried out by normalizing and segmenting logR values, and fitting a piecewise constant function to the data. logR changes were also used to infer approximate break points.

The method according to embodiments of the invention is advantageous over these prior art methods, since continuous informative PVAF-values are used. This has several advantages, including higher sensitivity for the reconstructed haplotypes since application of PVAF-values and subsequent segmentation alleviates the effect of stochastic WGA-artifacts, including incomplete ADO, ADI, preferential amplification (PA) as well as accounts for genuine copy number variants in the sample; the mosaic nature of few- or multi-cell DNA samples can be detected and dissected; not only mitotic trisomies and disomies can be discerned but also the meiotic and mitotic nature of the aberrations can be determined, furthermore copy neutral events such as UPhD and UPiD can be detected; moreover patches of LOH, which could be as a result of consanguinity, in each of the parents or the DNA of the individual under study can be detected. In particular, prior art methods do not allow to (simultaneously) determine copy number and haplotype using continuous polymorphic variant allele frequencies. This is especially complicated when using noisy genotyping data derived from samples comprising low amounts of genetic material, such as single-cell samples.

A need still exists for improved methods for haplotyping and/or copy number typing.

**Summary of the invention**

It is an object of the present invention to provide a computer-implemented method for the analysis of genetic material of a subject, said method comprising:

- obtaining continuous polymorphic variant allele frequency (PVAF) values of the genetic material of the subject;
- obtaining phased genotype information of a first parent and phased or unphased genotype information of a second parent;
- categorizing the continuous PVAF values in a first category corresponding to the first parent based on the genotype information of the first and second parent;
- subcategorizing the continuous PVAF values from the first category corresponding to the first parent into subcategories;
- segmenting said subcategorized PVAF values; and
- providing the segmented PVAF values to indicate a genetic anomaly in the genetic material of the subject and/or inheritance of the genetic material of the subject.

Also provided is a computer program product thereof, and a non-transitory machine-readable storage medium storing said computer program product. It is an advantage of the present invention to not use discrete PV-calls of a DNA-sample per se. This is important for single- or few-cell DNA-samples as the method of the invention alleviates the erroneous discrete PV-calls resulting from WGA-artifacts and/or algorithmic interpretation of the signals determined with the WGA-material.

It is an advantage of the present invention to have two inherent features: (1) parity feature within each parental profile and (2) complementarity feature between parental profiles.

It is an advantage of embodiments of the present invention to provide an improved method, based on polymorphic variant allele fractions, to genome-wide haplotype, copy-number profile and determine the mechanistic origins of DNA-anomalies in single- or multi-cell derived DNA samples.

This object is met by the method according to the independent claims of the present invention. The dependent claims relate to preferred embodiments.

In a first aspect the present disclosure provides a method for the analysis of genetic material of a subject, said method comprising:

- obtaining continuous polymorphic variant allele frequency (PVAF) values of genetic material of a subject;
- obtaining genotype information of a first parent;
- categorizing the continuous PVAF values in a category corresponding to the first parent based on the genotype information of the first parent;
- segmenting said categorized PVAF values; and
- providing the segmented PVAF values to indicate a genetic anomaly in the genetic material of the subject and/or inheritance of the genetic material of the subject.

In a related aspect the present disclosure provides a method for the analysis of genetic material of a subject, said method comprising:

- obtaining continuous polymorphic variant allele frequency (PVAF) values of genetic material of a subject;
- obtaining genotype information of a first and second parent;
- categorizing the continuous PVAF values in a category corresponding to the first parent based on the genotype information of the first parent and second parent;
- segmenting said categorized PVAF values; and
- providing the segmented PVAF values to indicate a genetic anomaly in the genetic material of the subject and/or inheritance of the genetic material of the subject.

In a second aspect, the present disclosure provides a method comprising

- obtaining continuous polymorphic variant allele frequency (PVAF) values of genetic material of a subject;
- obtaining phased genotype information of a first parent and phased or unphased genotype information of a second parent;
- categorizing the continuous PVAF values in a category corresponding to the first parent based on the genotype information of the first and second parent;
- subcategorizing the continuous PVAF values from the category corresponding to the first parent into subcategories;
- segmenting said subcategorized PVAF values; and
- providing the segmented PVAF values to indicate a genetic anomaly in the genetic material of the subject and/or inheritance of the genetic material of the subject.

In a third aspect, the present disclosure provides a method comprising:

- obtaining continuous polymorphic variant allele frequency (PVAF) values of the genetic material of the subject;
- obtaining phased genotype information of a first parent and phased genotype information of a second parent;
- categorizing the continuous PVAF values into a first category corresponding to the first parent and a second category corresponding to the second parent based on the genotype information of the first and second parent;
- subcategorizing the continuous PVAF values in the first and second categories into subcategories;
- segmenting said subcategorized PVAF values; and
- providing the segmented PVAF values to indicate a genetic anomaly in the genetic material of the subject and/or inheritance of the genetic material of the subject.

In a further aspect, the present disclosure provides methods for haplotyping and/or copy number typing genetic material of a sample, said method comprising:

- determining continuous polymorphic variant allele frequency (PVAF) values of the genetic material of the sample;
- providing phased parental polymorphic variant (PV) genotypes using genotypes of a close relative or the sample genotype itself;
- categorizing the determined continuous PVAF values of the genetic material of the sample using the provided (phased or unphased) parental PV genotypes, resulting in categorized PVAF values;
- subcategorizing said categorized PVAF values of the sample into subcategories using the provided parental phased PV genotypes;
- segmenting said subcategorized PVAF values, resulting in haplotyped / phased and segmented PVAF patterns.

In preferred embodiments of the invention the genetic material of the sample may be derived from a single cell, a few cells, a large number of cells or cell-free DNA. Continuous PVAF values have been determined using a sample comprising genetic material of the subject. In a particular embodiment, said sample comprises a low amount of genetic material of said subject, such as a sample comprising only one or a few cells of said subject, or a plasma sample obtained from a mother pregnant with said subject. In another particular embodiment, continuous PVAF values have been determined using an (in particular whole-genome) array or sequencing technology, especially the array and sequencing technologies as described herein. In yet another particular embodiment continuous PVAF values have been determined using a whole-genome amplified (WGAed) sample.
In preferred embodiments of the invention a polymorphic variant may be any genetic variant that has at least one alternative form when compared to a reference sequence. In a particular embodiment, said polymorphic variant is a single nucleotide polymorphism (SNP). In a further embodiment, said PVAF values are B-allele frequencies (BAFs).
In preferred embodiments of the invention, the method further may comprise normalizing DNA quantity values (such as read counts or logR values) of the genetic material according to the haplotyped / phased and segmented PVAF values, also referred to herein as (segmented) PVAF patterns. In particular, the method comprises obtaining DNA quantity values and normalizing said DNA quantity values based on said segmented PVAF values. Preferably, the DNA quantity values are logR values or read count values.
In preferred embodiments of the invention providing said phased parental PV genotypes comprises:

(a) phasing the parental PV genotypes based on the genotypes of a (close) relative, i.e. in a parent heterozygous at two syntenic loci, the designation of which allele at the first locus is on the same chromosome as which allele at the second locus.

In a particular embodiment, the method of the invention further comprises reflecting obtained PVAF values against the middle axis prior to segmentation. In a further preferred embodiment, the method of the invention comprises reflecting the obtained PVAF values, at a position corresponding to a specific phased parental genotype, against the middle axis prior to segmentation. Reflecting PVAF values provides a benefit as it improves segmentation (more PVAF values are present around a specific PVAF value for segmentation). In addition reflecting PVAF values aids in extracting haplotype information from continuous PVAF plots.

Preferably, reflecting PVAF values in the category corresponding to the first parent comprises:

- determining loci where said first parent has a particular phased genotype (e.g. either AB or BA); and
- reflecting PVAF values at determined loci around the middle axis.

In particular, reflecting the PVAF values is performed in both subcategories. In another preferred embodiment, the method also comprises reflecting PVAF values in the category corresponding to the second parent. It is to be noted that determining loci where PVAF values should be reflected in the first and second parental categories is not necessarily done for the same particular phased genotype. E.g. in the paternal category, PVAF values may be reflected for those loci where the paternal haplotype is AB; while in the maternal category, PVAF values may be reflected for those loci where the maternal haplotype is BA. However, within a specific parental category, reflecting of PVAF values is preferably performed at loci where said parent has a particular phased genotype (e.g. either AB for a biallelic marker).

In preferred embodiments of the invention, the method comprises:

(a) categorizing the determined continuous PVAF values of the genetic material of the sample in parental PVAF categories
(b) subcategorizing said parental PVAF categories of (a) and reflecting the determined PVAF values according to specific combinations of the parental phased PV genotypes.

In preferred embodiments of the invention the haplotyped / phased and segmented PVAF patterns provide an independent haplotype block call.

In preferred embodiments of the invention the parental scores and said haplotyped / phased and segmented PVAF patterns are used for normalizing DNA quantity values (herein also termed (relative) copy number-values) (e.g. logR) and determining the diploid chromosomes of the genetic materials of the sample.

In preferred embodiments of the invention, the method further may comprise integrating said normalized (relative) copy number values (e.g. logR) or a copy number profile with the haplotyped / phased and segmented PVAF patterns to reveal distinct signatures for different anomalies in the genetic material of the sample.

In a particular embodiment, categorizing the continuous PVAF values in a category corresponding to the first parent comprises:

- determining loci that are informative for the first parent using the genotype information of the first and optionally second parent; and
- categorizing continuous PVAF values of genetic material of the subject at said loci that are informative for the first parent in a category corresponding to the first parent.

In another particular embodiment, subcategorizing the continuous PVAF values from the category corresponding to the first parent comprises:

- determining loci with a specific genotype combination of the first and second parent;
- subcategorizing continuous PVAF values of genetic material of the subject at said loci with a specific genotype combination of the first and second parent.

In preferred embodiments of the invention, segmenting is performed using a segmentation method. The skilled person is aware of segmentation methods that are suitable to segment the (sub)categorized PVAF values, such as clustering segmentation methods including a K-means algorithm. In another particular embodiment, the segmentation method is a piecewise constant fitting algorithm. In yet another particular embodiment, the segmentation method is a binary segmentation method such as circular binary segmentation (CBS).

In preferred embodiments of the invention the method translates PVAF values of genetic material of the sample into haplotype blocks.

In another particular embodiment, indicating a genetic anomaly refers to indicating, in particular determining, the presence or absence of a genetic anomaly.

In preferred embodiments of the invention, the haplotyped / phased and segmented PVAF patterns affirm DNA copy-number and DNA copy-neutral anomalies.

In preferred embodiments of the invention, the haplotyped / phased and segmented PVAF patterns define the parental origin and mechanistic origin of numerical, structural or copy-neutral chromosomal anomalies.

In preferred embodiments of the invention the genetic material originates from a cleavage-stage or blastocyst-stage embryo.

In further preferred embodiments of the invention, the genetic material originates from a fetus or cell-free fetal samples.

In yet further preferred embodiments of the invention the genetic material originates from normal tissue and/or a cancer.

In preferred embodiments of the invention single-cell haplotyping preferably uses single-cell continuous PVAF-values (e.g. SNP B-allele frequencies or BAF). In this approach, instead of the single-cell discrete PV-genotype calls, single-cell continuous PVAF-values, are preferably exported from e.g. Illumina's GenomeStudio, which preferably subsequently are haplotyped / phased based on robust parental multi-cell PV genotypes, which in turn have been phased using a relative, such as e.g. a sibling or grandparental genotypes according to embodiments of the invention. Subsequently, consecutive single-cell PVAF-values are first split into four different subcategories (P1 and P2 in the paternal category; M1 and M2 in the maternal category) and flipped around the middle (0.5) axis as defined by phased informative PV-calls in the genotypes of the parents. Subsequently, these single-cell PVAF values are preferably segmented using for instance piecewise constant fitting. Applying this principle for maternal and paternal informative PVs separately results in independent PV-haplotype block calls. These haplotyped / phased and segmented single-cell PVAF patterns according to embodiments of the invention, not only independently confirm and improve single-cell haplotypes determined with discrete SNP-genotypes, but also indicate DNA-copy number and copy neutral DNA-anomalies. Furthermore, in further preferred embodiments, by integration with DNA-copy number profiles, they have the potential to discriminate genomic anomalies that are meiotic in origin from those that are mitotic in origin.

Embodiments of the present invention provide an innovative orthogonal method that can translate polymorphic variant allele frequencies (PVAFs) into haplotype blocks. Since PVAFs, which are continuous values rather than discrete AA, AB or BB calls enforced by a genotyping algorithm, are phased and interpreted according to embodiments of the invention, putative single-cell genotyping errors resulting from incomplete e.g. ADO or PA which derail standard phasing methods are advantageously accounted for. Hence, the segmented but haplotyped / phased PVAFs according to embodiments of the invention not only independently confirm the orthodox haplotypes computed from the discrete single-cell PV genotype calls, but also elegantly confirm DNA-copy number aberrations.

Preferred embodiments of the invention provide a method for cell-free, single-cell, few-cell or multi-cell DNA haplotyping, copy-number profiling, or discrete genotyping whereby said method uses polymorphic variant allele fraction (PVAF-) values and/or (relative) DNA copy number-values (e.g. logR) determined from DNA-samples analyzed with polymorphism typing platforms (e.g. SNP-arrays, next-generation sequencing including genome sequencing, exome sequencing and other targeted sequencing approaches). The DNA-samples may comprise whole-genome amplified DNA, partial genome amplification products and non-amplified DNA.

In further preferred embodiments the method may comprise the following steps:

- Massively parallel (genome-wide) typing of genetic polymorphism's allele frequencies in either of said DNA-samples (cell-free, single-cell, few-cell or multi-cell DNA; WGAed or not);
- Copy number typing using all genetic polymorphism's allelic frequencies across the entire genome or a selection thereof;
- Reconstructing the haplotype of the DNA-sample using all genetic polymorphic variant allelic frequencies across the entire genome or a selection thereof, whereby said reconstructing may comprise:

    i. Phasing the parental PV-genotypes based on a close relative or the discrete genotype of the sample itself;
    ii. Identifying informative PV-loci;
    iii. Categorizing PVAF data of studied DNA samples into two parental profiles, i.e. maternal and paternal profiles;
    iv. The parental PVAF data are reflected around middle (0.5) axis where the parents have BA PV-calls (or using similar approaches on AB PV-calls);
    v. Subcategorizing the parental PVAF data into four sub-profiles according to phased parental genotypes;
    vi. Segmenting each of the four sub-profiles, e.g. using Piecewise Constant Functions (PCF) or circular binary segmentation (CBS).
    vii. The PVAF segments in the cell-free, single-cell, few-cell or multi-cell DNA (WGAed or not) reveal inherited parental haplotypes.
    viii. PVAF-profile patterns reveal distinct signatures for different genomic anomalies and can be integrated with logR or (relative) DNA copy number profiles.

In preferred embodiments the polymorphic variant allele frequency (PVAF) values are haplotyped / phased based on parental polymorphic variant (PV) genotypes that have been phased using offspring or grandparental genotypes.

In further preferred embodiments the phased cell-free, single-cell, few-cell or multi-cell derived PVAF values are segmented using a segmentation algorithm, e.g. piecewise constant fitting (PCF) or circular binary segmentation (CBS).

In preferred embodiments the phasing and segmenting preferably culminates in PVAF-haplotype block calls.

In further preferred embodiments the phased and segmented PVAF-patterns affirm (relative) DNA-copy number and copy neutral DNA-anomalies.

In preferred embodiments the method further comprises integrating said (relative) DNA-copy number (e.g. logR-values) and the pattern of the haplotyped PVAF-values to discriminate the origin of each chromosomal anomaly as a result of a meiotic or mitotic non-disjunction event. In another embodiment, the present invention provides the methods described herein wherein the segmented PVAF values indicate the meiotic or mitotic origin of a genetic anomaly in the genetic material of the subject. In a further embodiment, the present invention provides those methods wherein the segmented PVAF values indicate the haplotype of the genetic material of the subject. In another further embodiment, the present invention provides those methods wherein the PVAF values indicate the copy number of a chromosome or chromosome region in the genetic material of the subject. In another particular embodiment, the segmented PVAF values indicate a homologous recombination site or a chromosomal breakpoint.

In further preferred embodiments the method further comprises integrating said (relative) DNA-copy number (e.g. logR-values) to discriminate the origin of each chromosomal anomaly as a result of meiotic I or meiotic II non-disjunction event. In preferred embodiments the trisomies that are meiotic I in origin can be discriminated from those that are mitotic or meiotic II in origin.

In further preferred embodiments the trisomies that are meiotic II in origin can be discriminated from those that are mitotic in origin, when at least one homologous recombination has occurred.

In preferred embodiments normal diploid chromosomes can be discriminated from uniparental isodisomy (UPiD) and uniparental heterodisomy (UPhD), and whereby UPiD can be discriminated from UPhD.

In preferred embodiments UPiDs that are meiotic II in origin can be discriminated from those that are mitotic in origin, when at least one homologous recombination has occurred.

Embodiments of the present invention advantageously provide an orthogonal method that translates cell-free, single-cell, few-cell or multi-cell PV allele frequencies into haplotype blocks.

By using the method according to embodiments of the inventions DNA copy neutral loss of heterozygousity (LOH) in each of the parents or the sibling can be found.

Advantageously embodiments of the method can be used to detect genetic mosaicisms in pools of few cells (WGAed or not), multi-cell (WGAed or not), or cell-free DNA-samples (WGAed or not), as well as their parental origin.

Advantageously embodiments of the method can be used to detect the allelic architecture of cell-free fetal DNA within the maternal blood stream.

Advantageously embodiments of the method can be used in non-invasive prenatal diagnosis (NiPD) for detecting the (im)balanced allelic architecture and haplotypes of the fetal genome.

Preferred embodiments of the invention provide methods for single-, few- or multi-cell genotyping, haplotyping, copy-number profiling and imputation of linked disease variants by determining the quantity, parental origin, as well as meiotic or mitotic origin of the alleles across the genome. More specifically preferred embodiments of the present invention relate to whole-genome polymorphic variant allele frequency (PVAF) analysis, as an innovative generic method for genetic diagnosis, where the genomic DNA can be derived from many cells, few cells, a single cell, or merely from cell-free DNA. The DNA is preferably processed by high throughput genotyping methods, e.g. SNP-arrays, next-generation sequencing. Furthermore, embodiments of the present invention advantageously are of paramount importance in understanding the etiology of genetic diseases, in particular genetic disorders with a Mendelian basis as well as genetic mosaicisms arising post-fertilization and de novo chromosomal anomalies by determining the allelic architecture of the genome. The present invention can therefore advantageously lead to major advancements in clinical settings and may lead to new insights into the mechanisms of genetic disorders.

In comparison to methods known in the art for determining copy number aberrations, parent-of-origin typing or haplotyping, a method according to preferred embodiments of the invention utilizes familial genotypes to retrieve informative PVAF-values and determines paternal and maternal haplotype-specific PVAF-profiles for the DNA-samples. Advantageously these DNA-samples can comprise not only conventional DNA-samples extracted from multiple cells, but also DNA-samples obtained of a single cell or a few cells requiring whole-genome amplification (WGA), a process known to introduce PVAF-errors due to allelic amplification artifacts, or DNA obtained from cell-free nucleic acids. Integration of the parental haplotype-specific PVAF-profiles and (relative) DNA copy number-values generates unique signatures. These signatures not only reveal genuine haplotypes, including the location of the parental meiotic homologous recombination sites, of the DNA-samples, but also decipher various types of chromosomal anomalies and their parental and mechanistic origin. Thus, a method according to embodiments of the invention also advantageously enables simultaneous haplotyping, traditional DNA copy-number typing, copy number typing of the haplotypes and parent-of-origin profiling of the DNA-samples.

Furthermore, the method of the invention facilitates the detection of genetic mosaicisms and their parental origin in few- or multi-cell DNA samples, following or not following WGA.

In a second aspect, the present invention relates to a computer program product comprising computer program code means adapted for performing all the steps of the method as described above, when the computer program product is run on a computer. In particular, the present invention provides a computer program product which is capable, when executed on a processing engine, to perform the methods described herein.

In a third aspect, the present invention provides a data carrier storing a computer program product according to the fourth aspect of the present invention. The term "data carrier" is equal to the terms "carrier medium" or "computer readable medium", and refers to any medium that participates in providing instructions to a processor for execution. Such a medium may take many forms, including but not limited to, non-volatile media, volatile media, and transmission media. Non-volatile media include, for example, optical or magnetic disks, such as a storage device which is part of mass storage. Volatile media include dynamic memory such as RAM. Common forms of computer readable media include, for example, a floppy disk, a flexible disk, a hard disk, magnetic tape, or any other magnetic medium, a CD-ROM, any other optical medium, punch cards, paper tapes, any other physical medium with patterns of holes, a RAM, a PROM, an EPROM, a FLASH-EPROM, any other memory chip or cartridge, a carrier wave as described hereafter, or any other medium from which a computer can read. Various forms of computer readable media may be involved in carrying one or more sequences of one or more instructions to a processor for execution. For example, the instructions may initially be carried on a magnetic disk of a remote computer. The remote computer can load the instructions into its dynamic memory and send the instructions over a telephone line using a modem. A modem local to the computer system can receive the data on the telephone line and use an infrared transmitter to convert the data to an infrared signal. An infrared detector coupled to a bus can receive the data carried in the infrared signal and place the data on the bus. The bus carries data to main memory, from which a processor retrieves and executes the instructions. The instructions received by main memory may optionally be stored on a storage device either before or after execution by a processor. The instructions can also be transmitted via a carrier wave in a network, such as a LAN, a WAN or the internet. Transmission media can take the form of acoustic or light waves, such as those generated during radio wave and infrared data communications. Transmission media include coaxial cables, copper wire and fibre optics, including the wires that form a bus within a computer.

In a particular embodiment, the present invention provides a non-transitory machine-readable storage medium storing a computer program product as described herein. In another particular embodiment, the present invention provides a non-transitory machine-readable storage medium storing the segmented PVAF values obtained by the method of the invention that indicate a genetic anomaly in the genetic material of the subject and/or inheritance of the genetic material of a subject.

Furthermore, the present disclosure provides a graphical user interface adapted for use of the methods of the invention.

In yet another particular embodiment, the present disclosure provides data structure or database of data structures for storing:

- genotype information of at least a first parent of a subject;
- continuous PVAF values of genetic material of said subject, wherein said continuous PVAF values have been categorized in a category corresponding to said first parent; and
- segmentation information for said categorized PVAF values.

In particular a data structure or database of data structures further adapted for use of the methods of the invention.

In a fourth aspect, the present disclosure provides in transmission of a computer program product according to the third aspect of the present invention over a network.

In preferred embodiments, the present disclosure provides the use of a method according to embodiments of the invention, for discovering the meiotic or mitotic nature of DNA aberrations in the genetic material of the sample.

Segmented PVAF values or patterns, according to embodiments of the invention, may be applied for haplotyping and/or DNA copy number variation detection and/or detecting copy number variation in haplotypes and/or parental origin detection of genetic material of a sample. Preferably said haplotyping, copy-number typing or parent-of-origin profiling comprises whole-genome screening of genetic material of a sample.

Embodiments of the present invention can advantageously be used as a generic approach for aneuploidy screening.

In a particular embodiment, the present disclosure provides a method to determine trisomy in the genetic material of a subject. In another particular embodiment, the present invention provides a method to determine the copy number of chromosome 13, 18 and/or 21. In a further embodiment, the copy number of chromosome 13. In another further embodiment, the copy number of chromosome 18. In yet another further embodiment, the copy number of chromosome 21. In another particular embodiment, the present invention provides a method to detect a genetic disorder selected from the group comprising Down syndrome, Edwards Syndrome, Patau Syndrome, Klinefelters syndrome, 47XXX, 47XYY, Turner syndrome, triploidy, DiGeorge syndrome, Cri du Chat syndrome, Angelman syndrome, Praeder-Willi syndrome, Wolf-Hirschhorn syndrome, Smith-Magenis syndrome, Williams-Beuren syndrome, Phelan-McDermid syndrome, Sotos Syndrome, Cystic Fibrosis, Muscular Dystrophy, Spinal Muscular Atrophy, Fragile X, Tay- Sachs disease, Gaucher disease, Torsion Dystonia, Niemann-Pick disease, Mucolipidosis, Fanconi Anemia, Canavan disease, Sickle Cell Anemia, Bloom Syndrome.

These and other aspects of the invention will be apparent from and elucidated with reference to the embodiment(s) described hereinafter.

**Brief description of the drawings**

Further features of the present invention will become apparent from the examples and figures, wherein:

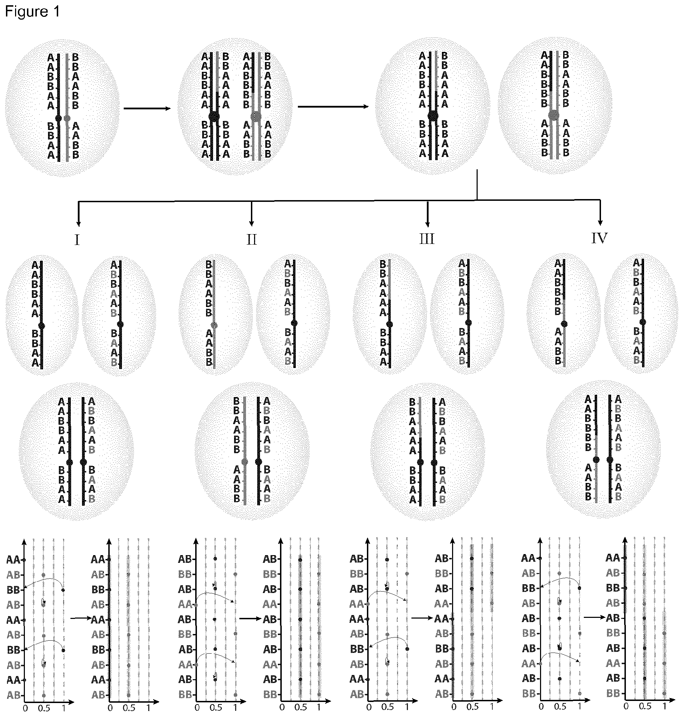

Fig. 1 illustrates the PVAF signatures of normal disomies (euploid) following PVAF analysis. The figure represents a pair of homologous chromosomes with one hypothetical homologous recombination event and the possible four resulting gamete compositions of the first parent are shown (I, II, III and IV). For simplicity we only consider 10 informative loci categorized in the first parental category to cover the plausible scenarios. After combination with a hypothetical gamete of a second parent (in the figure having haplotype ABBAABBAAB), the resulting cell is shown. Theoretical BAF values are plotted below and arrows indicate the flipping of particular BAF values. The BAF pattern that results after subcategorization and segmentation is shown directly next to the BAF plot. Dark grey segments indicate segmented BAF values that have been categorized in a first subcategory and light grey bars indicate segmented BAF values that have been categorized in a second subcategory.

Fig. 2 illustrates the PVAF signatures of monosomies following PVAF analysis. Moreover the figure represents a pair of homologous chromosomes with one hypothetical homologous recombination event. For simplicity we only consider 10 informative loci to cover the plausible scenarios.

Fig. 3 illustrates the PVAF signature of uniparental isodisomies (UPiD) following PVAF analysis. Moreover the figure represents a pair of homologous chromosomes with one hypothetical homologous recombination event. For simplicity we only consider 10 informative loci to cover the plausible scenarios.

Fig. 4 illustrates the PVAF signatures of uniparental heterodisomies (UPhD) following PVAF analysis. Moreover the figure represents a pair of homologous chromosomes with one hypothetical homologous recombination event. For simplicity we only consider 10 informative loci to cover the plausible scenarios.

Fig. 5 illustrates the PVAF signatures of a meiotic I trisomies following PVAF analysis. Moreover the figure represents a pair of homologous chromosomes with one hypothetical homologous recombination event. For simplicity we only consider 10 informative loci to cover the plausible scenarios.

Fig. 6 illustrates the PVAF signatures of meiotic II trisomies following PVAF analysis. Moreover the figure represents a pair of homologous chromosomes with one hypothetical homologous recombination event. For simplicity we only consider 10 informative loci to cover the plausible scenarios.

Fig. 7 illustrates the PVAF signatures of mitotic trisomies following PVAF analysis. Moreover the figure represents a pair of homologous chromosomes with one hypothetical homologous recombination event. For simplicity we only consider 10 informative loci to cover the plausible scenarios.

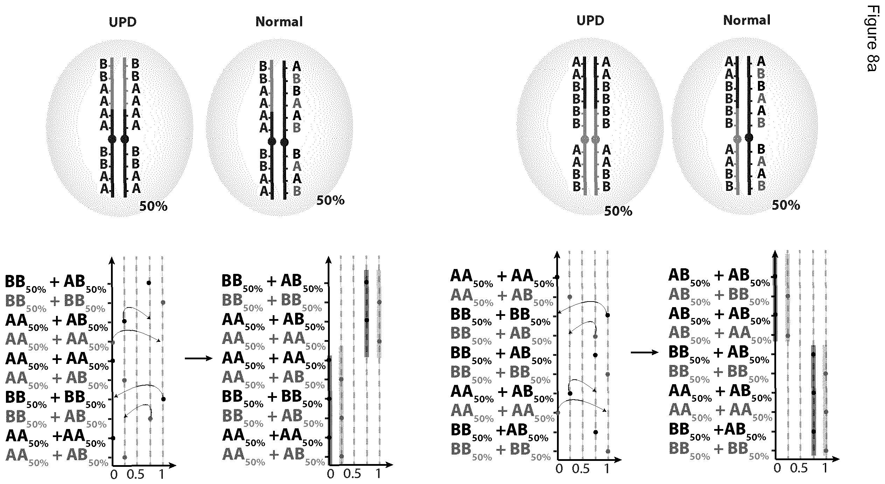

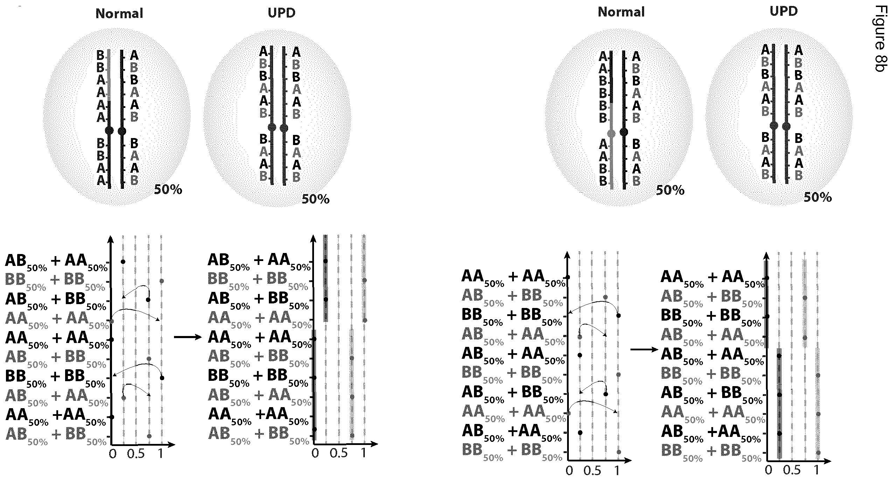

Fig. 8 illustrates the PVAF signatures of mosaic UPDs following PVAF analysis. Moreover the figure represents a pair of homologous chromosomes with one hypothetical homologous recombination event. For simplicity we only consider 10 informative loci to cover the plausible scenarios. If the mosaic UPD is maternal in origin, Fig. 8a shows the PVAF signatures in the maternal profile and Fig. 8b shows the PVAF signatures in the paternal profile. However, if the mosaic UPD is paternal in origin, Fig. 8a shows the PVAF signatures in the paternal profile and Fig. 8b shows the PVAF signatures in the maternal profile.

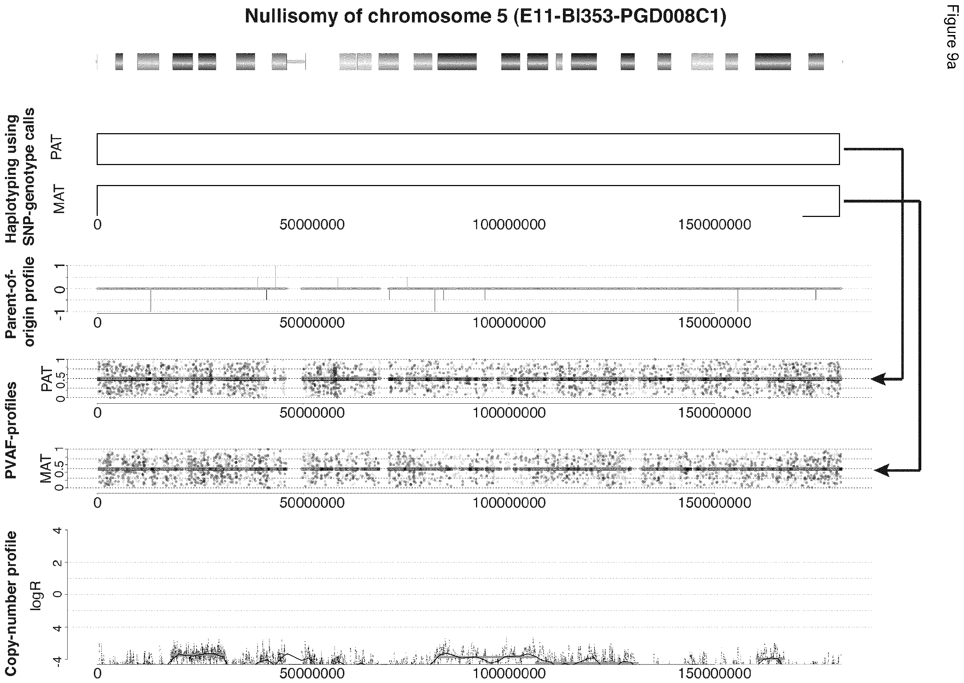

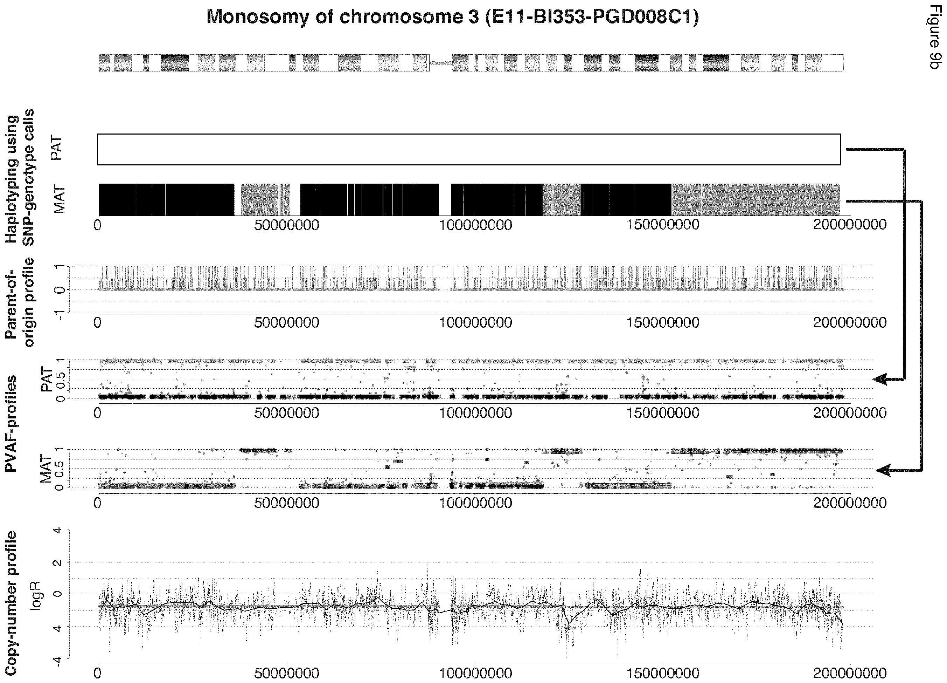

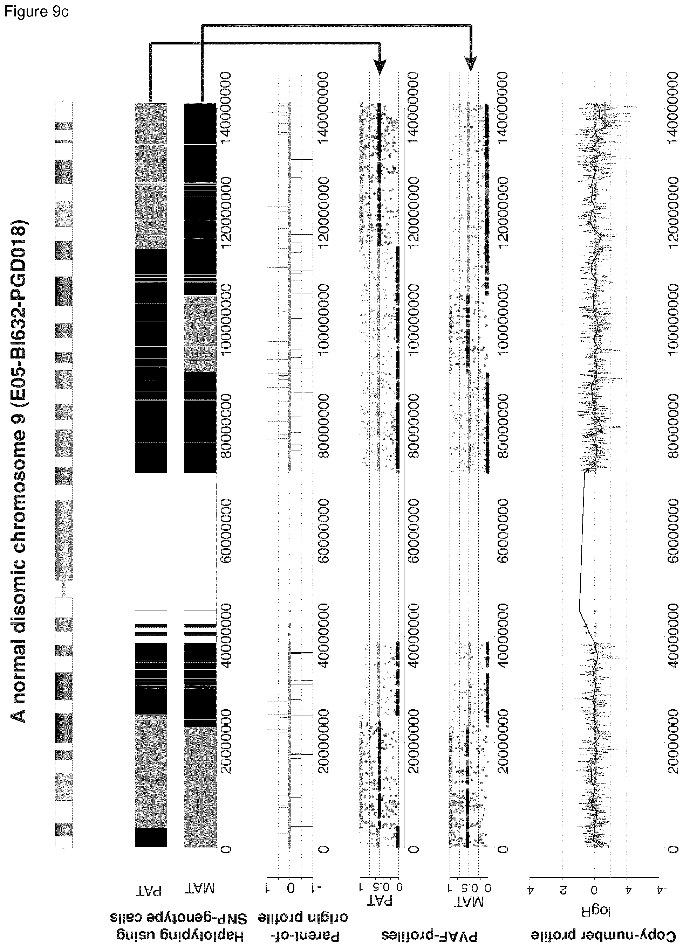

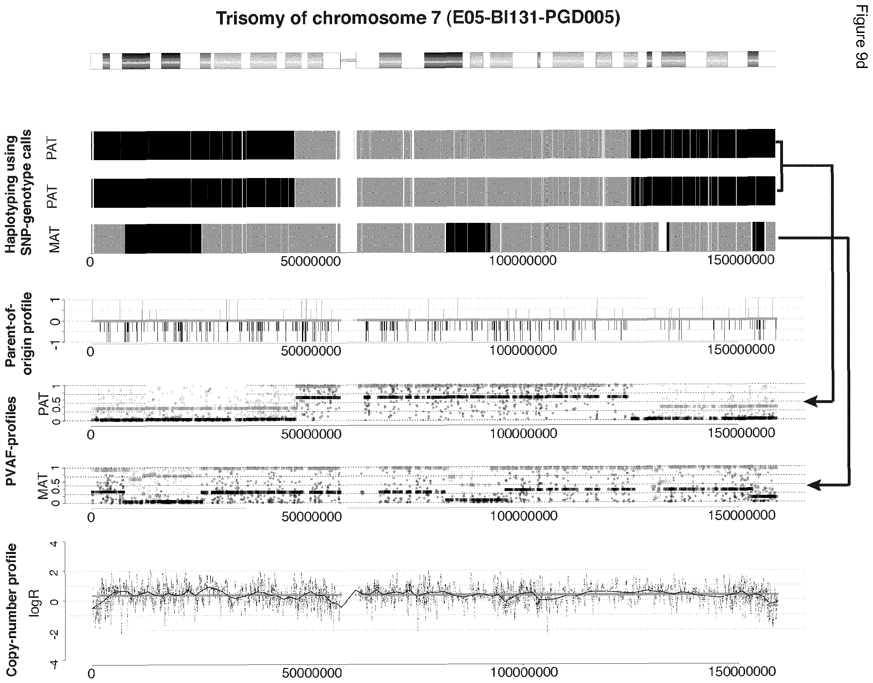

Fig. 9 illustrates different real data examples for full-chromosome aberrations detected by the present invention in single blastomeres. Fig. 9a depicts a nullisomy, Fig. 9b a maternal monosomy (i.e. maternal allele is retained), Fig. 9c a normal disomy and Fig. 9d a mitotic paternal trisomy (i.e. two copies of the paternal allele and one copy of the maternal allele).

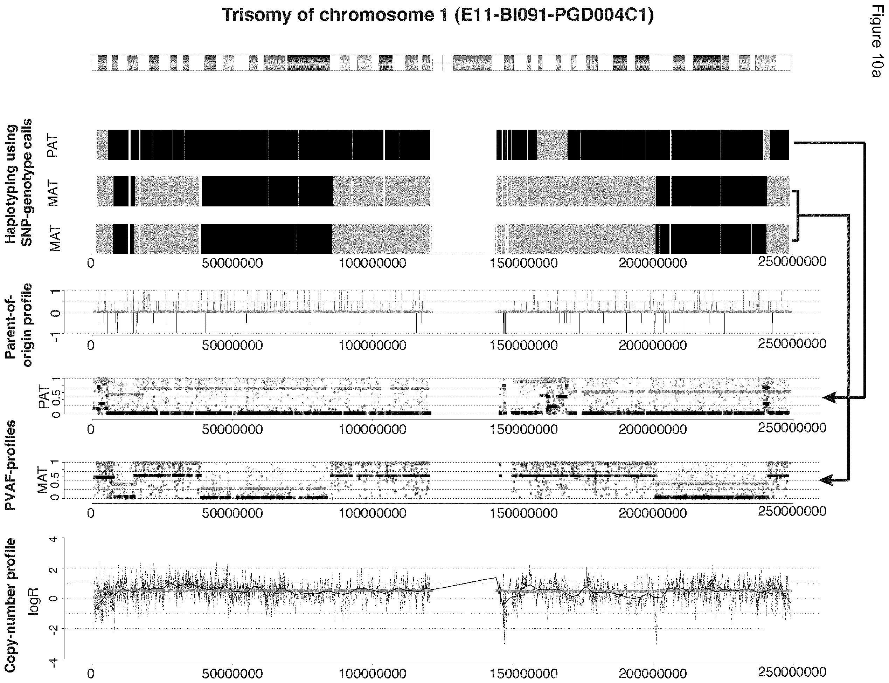

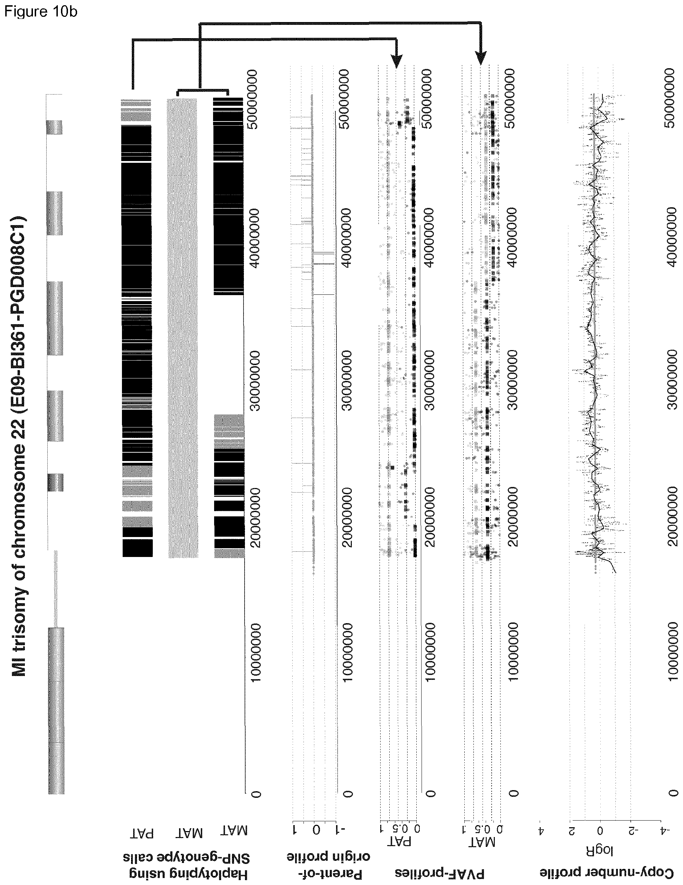

Fig. 10 illustrates different real data examples and demonstrates difference of meiotic and mitotic trisomies detected by the present invention in single blastomeres. Fig. 10a depicts a maternal mitotic trisomy while Fig. 10b represents maternal meiotic I trisomy.

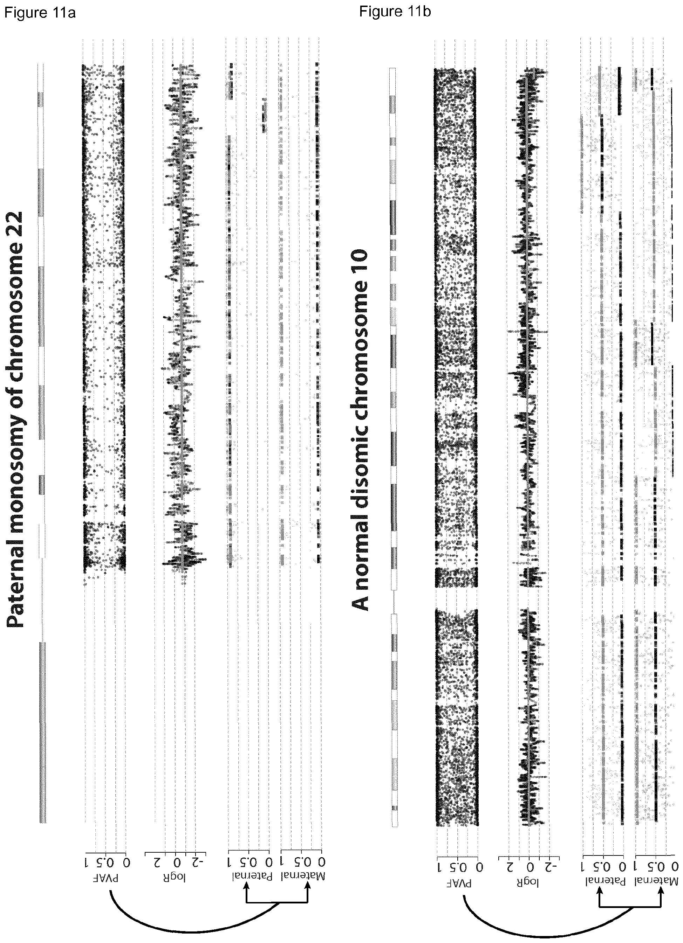

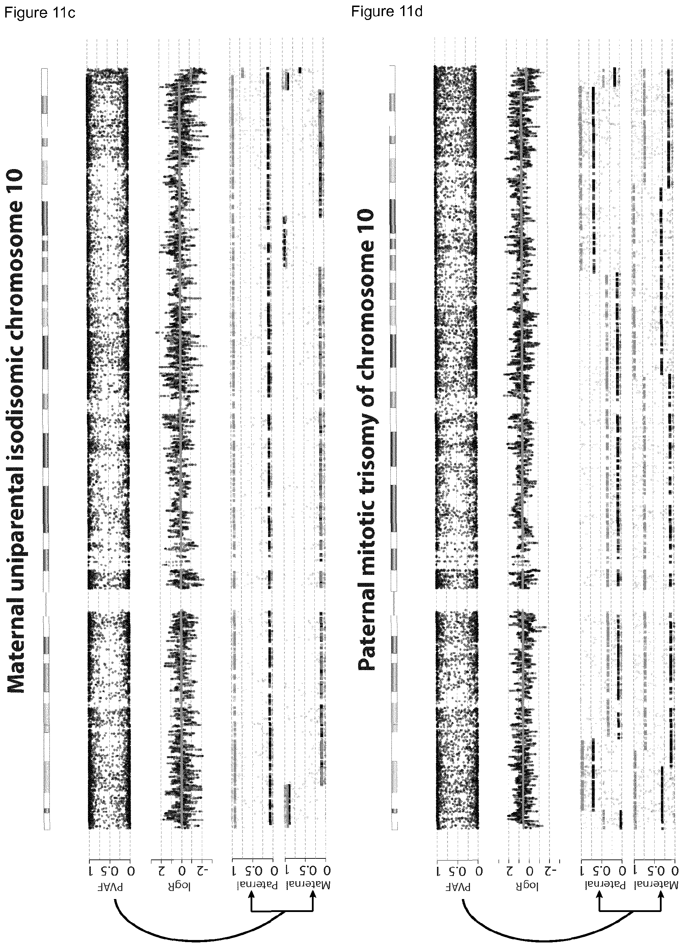

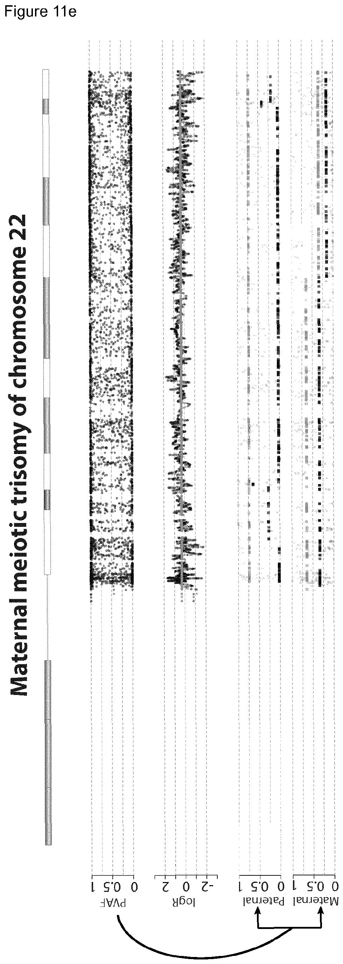

Figs. 11a-e illustrate parental haplotyped PVAF profiles used in embodiments of the invention, which illustrate the power of the embodiments of the present invention for detecting different chromosome abnormalities. Fig. 11a a paternal monosomy, Fig. 11b a normal disomy, Fig. 11c a numaternal UPiD, Fig. 11d a maternal mitotic trisomy, Fig. 11e a maternal meiotic trisomy.

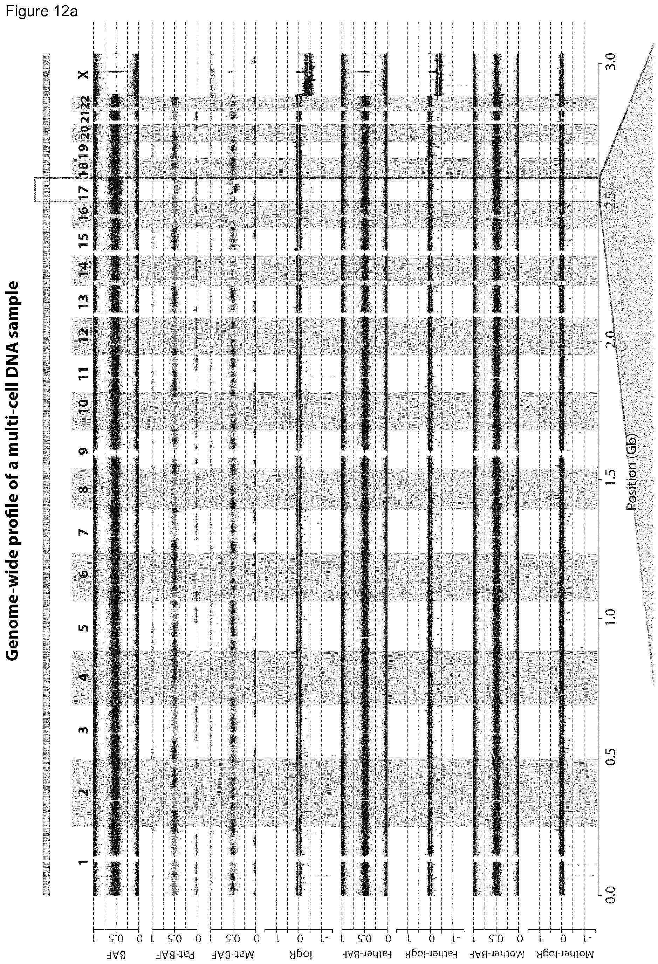

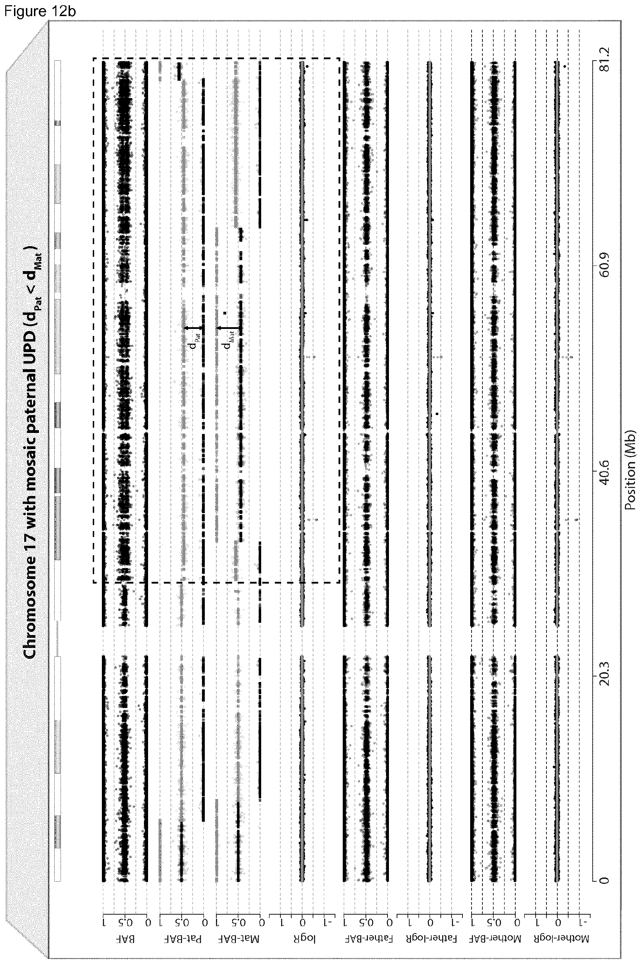

Figs. 12a and 12b illustrate a method according to embodiments of the invention for a multi-cell DNA sample having a chromosome 17q paternal UPiD mosaicism. Fig. 12a represents the whole-genome overview (chromosomes 1 to 22 and X), while Fig. 12b provides a more detailed view of chromosome 17.

Figs. 13a-c illustrates a flow chart, comprising a module according to a method according to embodiments of the invention.

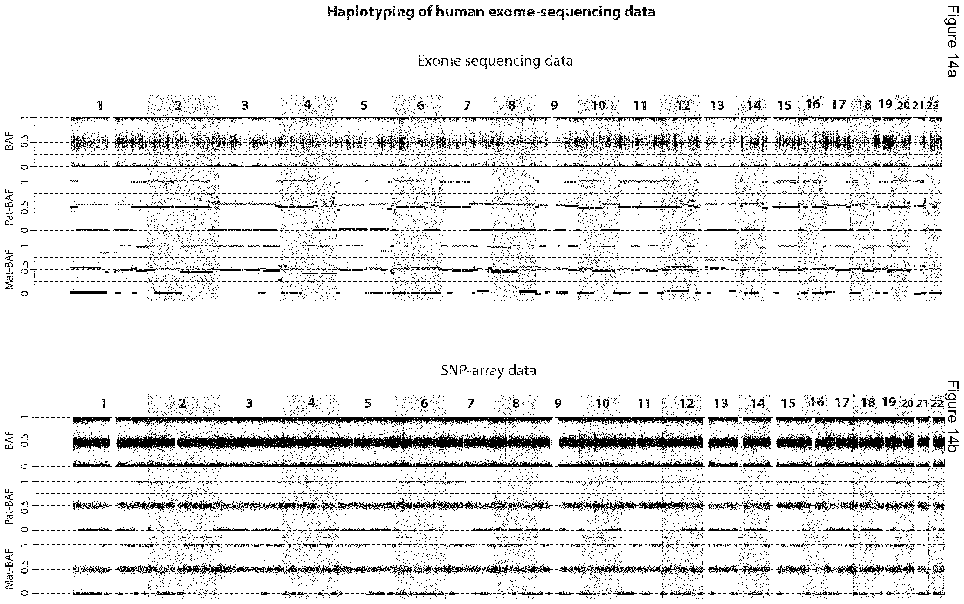

Fig. 14 illustrates the power of the present invention following sequencing of a reduced library representation of the human genome, e.g. exome sequencing. Fig. 14a depicts haplotyped PVAF profiles derived from exome sequencing data of a multi-cell DNA-sample.

Fig. 14b depicts haplotyped PVAF profiles derived from SNP-array data of the same DNA-sample. The comparison of the haplotyped PVAF profiles following exome sequencing (Fig. 14a) and SNP-array (Fig. 14b) shows the application of the present invention on sequencing data.

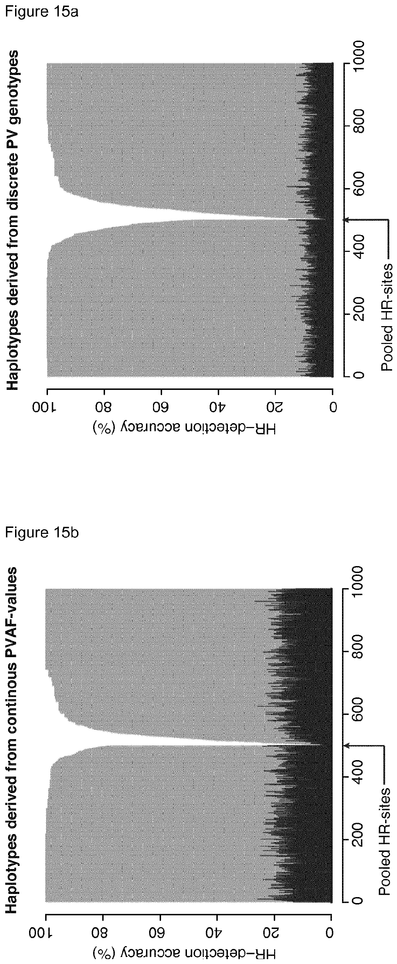

Fig. 15 illustrates the accuracy of homologous recombination site (HR-site) detection after transformation of haplotyped / phased and segmented PVAF parental profiles to discrete parental haplotypes. Fig. 15a depicts the accuracy (%) of the PVAF transformed single-cell haplotypes deduced from PVAF-values of 5 EBV-transformed single cells that are matched to the correct haplotype in comparison with their multi-cell haplotype reference. The single-cell haplotype concordance frequencies are depicted for 500 SNPs up- and downstream of HR-sites present in two individuals. Fig. 15b depicts the accuracy (%) of the single-cell haplotypes deduced form the discrete SNP-calls of the same 5 EBV-transformed single cells that are matched to the correct haplotype in comparison with their multi-cell haplotype reference. The single-cell haplotype concordance frequencies are depicted for 500 SNPs up- and downstream of HR-sites present in two individuals. The raw haplotypes are shown in back and the interpreted haplotypes are shown in grey.

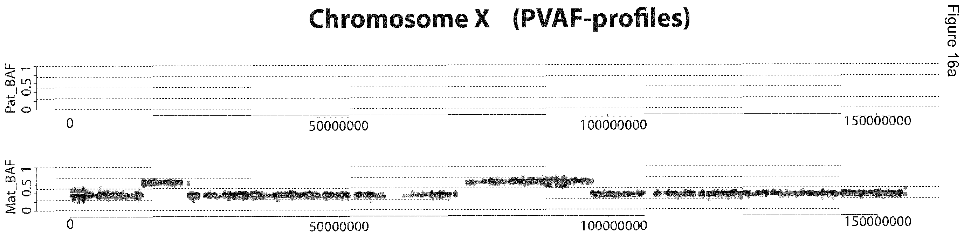

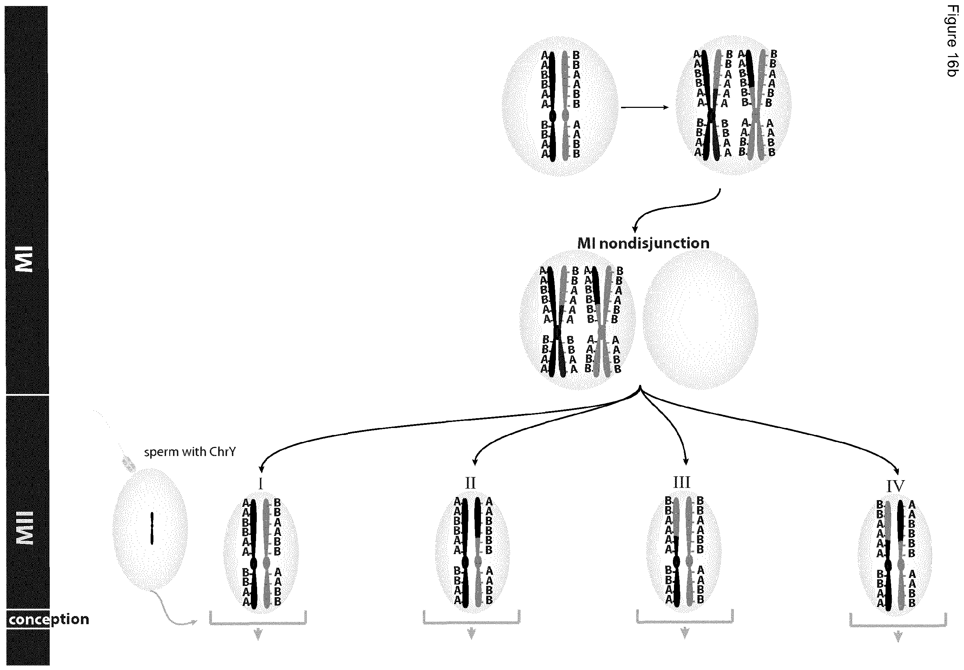

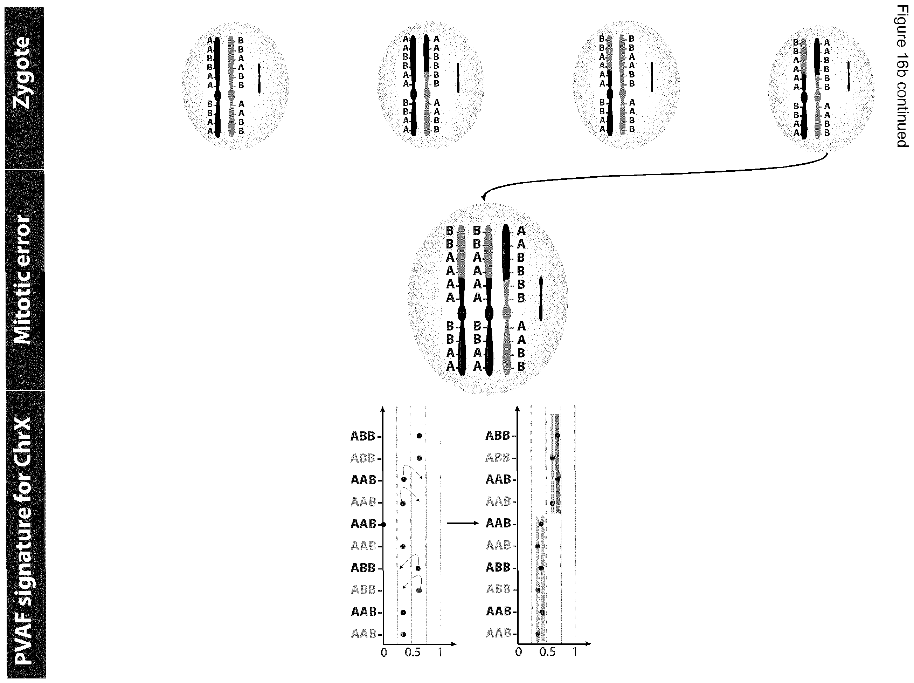

Fig. 16 illustrates a multi-cell DNA-sample with XXXY karyotype caused by both meiotic I and mitotic errors. Fig.16a demonstrates haplotyped PVAF profiles of chromosome X deduced form SNP-array data of this DNA-sample. Fig.16b demonstrates the schematic representation of how the aberration traced back to errors that were occurred at both meiosis I and mitosis of this individual.

Fig. 17 provides a schematic overview of the method of the invention according to a preferred embodiment. Paternal genotype information is indicated with squares, while maternal information is indicated with circles.

The drawings are only schematic and are non-limiting. In the drawings, the size of some of the elements may be exaggerated and not drawn on scale for illustrative purposes. Any reference signs in the claims shall not be construed as limiting the scope. In the different drawings, the same reference signs refer to the same or analogous elements.

**Detailed description of preferred embodiments**

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

In the drawings, like reference numerals indicate like features; and, a reference numeral appearing in more than one figure refers to the same element. The drawings and the following detailed descriptions show specific embodiments of a method or device for haplotyping and/or copy number typing genetic material, more specifically methods or devices for haplotyping, copy number typing, and genotyping by polymorphic variant allele frequency analyses.

Where in embodiments of the present invention reference is made to a **"polymorphic variant"** (PV), reference is made to polymorphic variants, which may be bi-allelic or multi-allelic genetic variants segregating in a family or population, without any specific cutoff on the minor allele frequency in the population. **"Discrete PV genotypes or PV-calls"** refer to distinct values representing PVs. These values could be descriptive (e.g. in the case of bi-allelic PV: 'AA', 'AB' and 'BB'; or the base genotypes 'AT', 'CG','TG','AA' etc.) or numerical values (e.g. in the case of bi-allelic PV genotypes are encoded as: '11', '10' and '00'). **"Continuous PV-values"** refer to numerical values that are not restricted to distinct values and could be in a continuous range between two consecutive numerical discrete values. These values can have decimals.

Where in embodiments of the present invention reference is made to **"polymorphic variant allele frequency"** (PVAF) or "polymorphic variant allele fraction", reference is made to the fraction of one allele over the total amount of alleles in the DNA sample following genotyping. Conventionally, for biallelic PVs, PVAF refers to B-allele frequency (BAF) that is the fraction of B-alleles in the PV-typing data, which may be obtained from a DNA-sample by high throughput genotyping methods, e.g. SNP-arrays or next-generation sequencing technologies. In a preferred embodiment, a polymorphic variant allele frequency is a B-allele frequency. Evidently, when a claim or embodiment of the invention refers to a B-allele frequency, A-allele frequencies could be used as well. B-allele frequencies comprise A-allele frequency information and vice versa.

In general, a PVAF value is expressed using a value from 0 to 1, as they refer to the frequency or fraction. In principle, PVAF values may be expressed using a multiplicity of said value, e.g. using a value from 0 to 100. For example, a PVAF value of 0.5 that indicates that half of total amount of alleles has the polymorphic variant allele, may be expressed as e.g. 50. In that instance, a PVAF value of 1 (i.e. all alleles have the particular genotype) will be expressed as 100. As referred to herein, $PVAF_{max}$ indicates the maximal PVAF value (i.e. all alleles have the particular genotype) and $PVAF_{min}$ indicates the minimal PVAF value (i.e. none of the alleles have the particular genotype). Throughout the present application, PVAF (in particular BAF) values are indicated using a value from 0 to 1, thus $PVAF_{min}$ being 0 and $PVAF_{max}$ being 1. Nonetheless, embodiments of the invention are not restricted to PVAF values expressed using this particular range. Furthermore, the middle axis of PVAF values refers to the axis running through the values indicating that half of the total amount of alleles has the polymorphic variant. Therefore, the middle axis is referred to herein also as the 0.5 axis. When using e.g. a PVAF value range of 0 to 100, the middle axis refers to the axis running through 50, etcetera. In particular, throughout the present application, if a PVAF value of 1 is mentioned, this refers to $PVAF_{max}$. If a PVAF value of 0.5 is mentioned, this refers to the middle (median) PVAF value (i.e. the middle of $PVAF_{min}$ and $PVAF_{max}$).

Reflecting a value against the middle axis (also referred to as 'mirroring' or 'flipping') refers to a process wherein values are reassigned the value of the reflection of the value over the middle axis. When PVAF values are expressed using a value from 0 to 1, the 0.5 axis being the middle axis, exemplary reflected values are as follows:

| Original PVAF value | Reflected PVAF value |
|---|---|
| 0 | 1 |
| 0.3 | 0.7 |
| 0.5 | 0.5 |

(continued)

| Original PVAF value | Reflected PVAF value |
|---|---|
| 0.8 | 0.2 |
| 1 | 0 |

Where in embodiments of the present invention reference is made to **high throughput genotyping** technologies, reference is made to any massively parallel sequencing, SNP-array or next-generation sequencing technologies. A high throughput genotyping technology provides, after initial processing, raw PV-data, including genotype calls, DNA quantity values and PVAF-values.

**"DNA quantity values"** refer to high throughput genotyping measurements that indicate the quantity of genetic material present in the sample. Typical DNA quantity values obtained using SNP-array genotyping are logR values. Typical DNA quantity values obtained using sequencing technologies are read count and/or logR values. DNA quantity values may have been determined locally or genome-wide. Preferably, genome-wide DNA quantities are obtained and used or normalized in the methods of the present invention. Normalization of DNA quantity values refers to a process that normalizes the raw DNA quantity values (e.g. logR-values). In general, this procedure can consist of: (1) correction for %GC-bias in the raw values, (2) detection of the likely normal disomic chromosomes, and (3) trimmed mean (or median) correction on the basis of detected normal disomic chromosomes (see the detailed description of the present invention).

Where in embodiments of the present invention reference is made to (massively parallel) **sequencing** technologies, reference is made to any next-generation sequencing methodology, e.g. whole-genome sequencing, exome sequencing, targeted sequencing.

**Whole genome amplification (WGA):** WGA is a process that is applied to DNA to increase the amount of DNA. Single-cell and few-cell DNA-samples may require WGA to produce enough input DNA for high-throughput genotyping technologies. Different WGA methods have been described and are typically based on either multiple displacement amplification (MDA) or a PCR-based genome-wide amplification or a combination thereof. WGA is known to produce different kinds of artifacts, including amplification bias according to genome base composition (e.g. %GC-content), allele drop-out (ADO), allele drop-in (ADI), preferential amplification (PA), chimeric DNA molecules and nucleotide copying errors.

**Genotype information:** Genotype information of a parent refers to the genetic makeup of the parent. Genotype information may be unphased or phased genotype information. Parental genotype information may be obtained directly by genotyping a sample obtained from the parent (e.g. by massive parallel sequencing or array-typing of a parental blood or tissue sample). In other instances, genotype information of the parent of a subject may be obtained indirectly, e.g. by genotyping close relatives (such as siblings and/or grandparents). Subsequently, the genotype information of the parent may be derived from the available genotypes of the close relatives. The skilled person is aware on how to compute a genotype based on genotypes of close relatives. Preferably, the genotype information of the parent(s) is available as discrete polymorphic values.

**Phased genotype:** Phasing of (PV) genotypes of the parent(s) can be attained by the use of a close relative, e.g. grandparents and/or a sibling and/or one or a few embryos. In the phasing process the close relative is used for phasing to determine the order of heterozygous PV genotypes on the parental alleles.

**'Informative loci' or 'Informative PV loci':** Informative loci refer to loci where the parental genotypes allow to positively identify from which parent the alleles in the subject originate from. In general, informative loci as used herein are the ones where one parent has homozygous PV genotypes and for the same loci the other parent has heterozygous PV genotypes. Nonetheless, semi-informative loci wherein one parent is homozygous and another parent is homozygous for a different allele, may also be used in the methods of the invention. For example, if the first parent has genotype AA and the second parent has genotype BB, an AB genotype in the subject identifies the paternal original of the first (A, originating from first parent) and second (B, originating from second parent) allele. Semi-informative loci are in particular useful for additional parent-of-origin typing or for confirming the parent-of-origin determined using informative loci (homozygous-heterozygous combination) only.

**Parent:** In the context of the present invention, a parent refers to a biological parent of the subject. In the instance of a so-called three-parent embryo/fetus/child, wherein two parents contribute to the chromosomal DNA and a third party contributes the mitochondrial DNA, a parent refers to a parent contributing to the chromosomal DNA.

Where in embodiments of the present invention reference is made to a sample comprising genetic material (in particular a DNA sample), reference is generally made to all samples comprising genetic material derived from: a single cell, a few cells, a large-number of cells or cell-free DNA; whole-genome amplified (WGAed) on non-WGAed. A sample comprising genetic material may also refer to a cell-free sample obtained from a body fluid sample.

Where in embodiments of the present invention reference is made to a multi-cell (DNA) sample, reference is generally made to DNA samples derived from different specimens with fixed representation of DNA among all cells or admixture of cells with mosaic architecture. The latter could be consisting of normal and abnormal admixture of cells, e.g. mosaic mixture of normal and aberrant cells in tumor specimens.

As is evident from the description of the invention herein, the methods of the present invention are preferably applied to samples containing low amounts of target nucleic acids, also referred to as genetic material. In particular, said genetic material of interest is either present within one or a few target cells, or as free circulating material in the sample. Thus in a particular embodiment, said sample contains one or a few target cells. In a further embodiment, said sample contains one target cell. In another embodiment, said sample contains a few target cells, in particular 1 to 30, more in particular 1 to 20, target cells. For example, 1-15, 1-10, 1-8, 1-7, 1-6, 1-5, 1-4, 1-3, one or two target cells. In another particular embodiment, target nucleic acids are present in an amount of 2 ng or less in said sample, in particular 1 ng or less, more in particular 0.5 ng or less. In another particular embodiment, target nucleic acids are present in an amount of 250 pg or less in said sample; in particular 200 pg or less; more in particular 150 pg or less. In another particular embodiment, said target nucleic acids are present in an amount of 100 pg or less; in particular in an amount of 50 pg or less; more in particular in an amount of 30 pg or less. In another particular embodiment, said target nucleic acids are cell-free, circulating nucleic acids. For example, circulating cell-free fetal DNA from a maternal sample, or circulating tumor DNA from a patient sample. While genetic material (e.g. maternal DNA) may be abundant in such samples, target DNA (i.e. genetic material of the subject to be researched, e.g. fetal DNA) is present in only very limited amounts. In a particular embodiment, target nucleic acids are present as cell-free nucleic acids in a fluid sample. In particular, said cell-free nucleic acids are present in a fluid sample comprising additional (non-target) nucleic acids. In a particular embodiment, said sample comprises a mixture of target and non-target nucleic acids. Preferably, said target nucleic acids are present in an amount between 0.1 and 20% of said non-target nucleic acids. In another particular embodiment, said sample comprises a mixture of target and non-target nucleic acids, wherein said target nucleic acids are present in an amount of 700 ng or less, in particular 500ng or less, more in particular 300 ng or less. In a further embodiment, 200 ng or less, in particular 100 ng or less, more in particular 50 ng or less. In yet another embodiment, said sample comprises cell-free nucleic acids, wherein said cell-free nucleic acids are present in an amount as defined hereinabove.

In a particular embodiment, providing a sample comprising low amounts of target nucleic acids comprises isolating one or a few target cells. The methods of the invention may further comprise lysing one or a few target cells.

A **"single-cell (DNA) sample"** refers to a (DNA) sample that is derived from a solitary cell of any tissue or cell type. Since a single cell only contains a few picogram of DNA, methods involving single-cell samples preferably comprise (whole genome) amplification for genotyping of the polymorphic variants. **"Few-cell (DNA)**

sample" refers to a (DNA) sample that is derived from a few cells of any tissue or cell type. Depending on the number of cells used for DNA extraction, methods involving such a sample may comprise (whole genome) amplification for genotyping of the polymorphic variants. **"Multi-cell (DNA) sample:** a DNA sample that is derived from a large number cells of any tissue or cell type.

**"Cell-free (DNA) sample"** refers to a sample that is derived from a fluid specimen that contains circulating genetic material. This can refer to cell-free fetal DNA that freely circulates in the maternal blood stream (also called free fetal DNA, ffDNA). An example of such a sample is a body fluid sample (in particular a blood, plasma or serum sample; more in particular a plasma sample) obtained from a pregnant female and comprising a mixture of maternal and fetal genetic material. Another embodiment of a cell-free sample refers to cell-free tumor DNA that freely circulates in the patient's blood stream.

The sample is preferably obtained from a eukaryotic organism, more in particular of a mammal. In a further preferred embodiment, said sample is from non-human animal (hereinafter also referred to as animal) origin or human origin. In a particular embodiment, said animal is a domesticated animal or an animal used in agriculture, such as a horse or a cow. In a further particular embodiment, said animal is a horse. In another particular embodiment, said sample is of human origin. In yet another particular embodiment, said sample is obtained from a pregnant woman. In another embodiment, said sample is obtained from a patient suspected from having a tumor or cancer. In another particular embodiment, said cell is a eukaryotic cell, in particular a mammalian cell. In a more particular embodiment, the origin of said cell is as described according to preferred embodiments regarding the sample origin as described above. In another particular said target nucleic acids are of eukaryotic origin, in particular of mammalian origin. In a more particular embodiment, said target nucleic acids are as described according to the preferred embodiment regarding the sample origin. Relating thereto, in a preferred embodiment, said target nucleic acids originate from an embryo or a fetus. In another preferred embodiment, said target nucleic acids originate from a (suspected) cancer or tumor cell.

The methods of the invention are applicable on any cell type. Preferred cells are polar bodies, blastomeres, trophectoderm cells from blastocysts or chorionic villus samples. Preferred genetic material comprises DNA, more particularly cell-free DNA. Preferably the cell-free fetal DNA is from maternal blood, plasma or serum. Both intact fetal cells and fetal cell-free nucleic acids (DNA, RNA) can be identified in maternal blood. The primary source of most fetal cell-free nucleic acids in the maternal circulation is thought to be apoptosis of placental cells. As already mentioned hereinbefore, the methods are applied on a small number of these cell types, i.e. on a few cells, in particular up to 50 cells; more in particular selected from 1, 2, 3, 4, 5, 6, 7, 8, 9, 10 or more cells up to 30; even more in particular on one or two cells. When applied on trophectoderm said few cells may be selected from 1, 2, 3, 4, 5, 6, 7, 8, 9, 10 or more cells; in particular up to 50 trophectoderm cells.

For the removal of the appropriate at least one cell, the zona pellucida at the cleavage and blastocysts stages can be breeched by mechanical zona drilling, acidified Tyrodes solution or laser. In preferred embodiments of the invention at least one, preferably a single cell, is a human or animal blastomere. In particular embodiments, the genetic testing is applied for diagnostic testing, carrier testing, prenatal testing, preimplantation testing, or predictive and presymptomatic testing. In these particular embodiments genetic testing assists to help patients achieve success with assisted reproduction. In another particular embodiment, the methods of the invention are applied for newborn screening. In yet another particular embodiment, the methods of the invention are applied for forensic testing.

"Genome-wide" as used herein means that the methods are applied to and provide information on sequences throughout the genome. In particular, the methods of the present invention provide information regarding all chromosomes for which at least fragments are present in the sample. In a particular embodiment, "genome-wide" refers to information regarding at least one variant per 100 Mb, in particular at least one variant per 10 Mb, in particular at least one variant per 1Mb throughout the genome. In a further embodiment, it is meant at least one variant per window of 100 Mb, in particular at least 1 variant per window of 50 Mb, more in particular at least one variant per window of 10 Mb throughout the genome. In another particular embodiment, genome-wide refers to information regarding at least one variant per window of 1 Mb. In a further embodiment, it is meant at least one variant per window of 100 kb, in particular at least 1 variant per window of 50 kb, more in particular at least one variant per window of 10 kb throughout the genome. In yet another embodiment, genome-wide refers to information regarding at least one variant per window of 1 kb.

"Genetic anomaly" refers to any abnormality in the genome. Genetic anomaly detection may involve detecting the presence or absence of genetically normal or abnormal chromosomes or chromosome regions. Genetic anomalies may be detected directly or indirectly. Direct detection of anomalies e.g. comprises direct detection of missing, extra, or irregular portions of chromosomal DNA. Indirect detection of anomalies comprises determining obtaining inheritance information to detect if a genetic anomaly has been inherited by a subject. For example, the methods of the present invention allow to determine if a genetic defect in one or more chromosome fragments of a father or mother have been inherited by an embryo, a fetus or a child. As detailed herein, indicating a genetic anomaly may also comprise indicating the meiotic or mitotic origin of said anomaly. In another embodiment, indicating a genetic anomaly refers to providing a map showing the copy number of a chromosome or chromosome region. In a further embodiment, said map covers a whole chromosome. In an even further embodiment, said map covers all autosomes, in particular all chromosomes.

'Inheritance" of genetic information refers to any information regarding the inheritance of genetic information from parents or ancestors. In particular, inheritance refers to the distribution of genetic information within an extended family (i.e. one or more parents, grandparents, siblings, nephews, nieces, aunts, uncles, and/or nieces). In a particular embodiment, the present invention provides a method to determine from which parent and/or grandparent a chromosome region has been inherited. In a further embodiment, indicating inheritance of genetic information refers to providing the haplotype of a chromosome or chromosome region. In another embodiment, indicating inheritance refers to providing a map showing which chromosome or chromosome region has been inherited from which relative. In a further embodiment, said map covers a whole chromosome. In an even further embodiment, said map covers all autosomes, in particular all chromosomes. Inheritance information not necessarily comprises information regarding genetic anomalies. For example, the present invention allows constructing haplotypes and/or determining inheritance of genomes without genetic defects within a family.

**Paternal allelic distance ($d_{Pat}$):** $d_{Pat}$ is a local distance between segmented P1 and P2 values.

**Maternal allelic distance ($d_{Mat}$):** $d_{Mat}$ is a local distance between segmented M1 and M2 values.

**Parity feature:** following segmentation the resulting subcategorized segments in a parental category (e.g. P1 and P2, or M1 and M2) have approximately the same length.

**Complementarity feature:** following segmentation the vertical distance (also referred to as allelic distance) between the resulting subcategorized segmented PVAF-values in a first parental category change in a complementary manner with the vertical distance (allelic distance) between the resulting subcategorized segmented PVAF-values in the other parental category.

**Degree of mosaicim ($\rho$):** p denotes the proportion of abnormal cells in a mosaic DNA-sample. (1-p) denotes the proportion of genetically normal (euploid) cells in a mosaic DNA-sample.

In preferred embodiments of the invention, a method is provided which is based on a concept that is applied to define preferably (a) the haplotypes of alleles in a cell-free, single-cell, few-cell or multi-cell DNA sample (either following WGA or not), (b) the copy number state of alleles or haplotypes in a cell-free, single-cell, few-cell or multi-cell DNA sample (either following WGA or not), (b) the parental and putative mechanistic origin of allelic anomalies in a cell-free, single-cell, few-cell or multi-cell DNA sample (either following WGA or not).

Preferably, categorizing the continuous PVAF values in a category corresponding to a first parent comprises:

- determining loci that are informative for the first parent using the genotype information of the first and second parent; and
- categorizing continuous PVAF values of genetic material of the subject at said loci that are informative for the first parent in a category corresponding to the first parent.

In particular, loci that are informative for the first parent comprise loci for which the first parent is heterozygous and for which the second parent is homozygous.

It is to be noted that throughout the present application, categorized PVAF values are also referred to as paternal or maternal PVAF values if they have been categorized in the paternal or maternal category, respectively. Thus, as used herein a paternal PVAF value refers to the sample's PVAF value in the paternal category.

In a further particular embodiment, subcategorizing the continuous PVAF values from the category corresponding to the first parent comprises:

- determining loci with a specific genotype combination of the first and second parent;
- subcategorizing continuous PVAF values of genetic material of the subject at said loci with a specific genotype combination of the first and second parent.

In another further particular embodiment, subcategorizing the continuous PVAF values from the category corresponding to the first parent comprises:

- determining loci where the first allele of the first parent comprises a genotype that is identical to the genotype of the two alleles of the second parent and subcategorizing continuous PVAF values at said loci in a first subcategory; and
- determining loci where the second allele of the first parent comprises a genotype that is identical to the genotype of the two alleles of the second parent and subcategorizing continuous PVAF values at said loci in a second subcategory.

It is to be understood that in the embodiments of the present invention, the subcategorization can be inversed in which case subcategorizing the continuous PVAF values from the category corresponding to the first parent may comprise :

- determining loci where the genotype of the first allele of the first parent comprises a genotype that is different from the genotype of the two alleles of the second parent and subcategorizing continuous PVAF values at said loci in a first subcategory; and
- determining loci where the second allele of the first parent comprises a genotype that is different from the genotype of the two alleles of the second parent and subcategorizing continuous PVAF values at said loci in a second subcategory

In a particularly preferred embodiment, the present invention provides a method comprising:

- obtaining continuous polymorphic variant allele frequency (PVAF) values of the genetic material of the subject;
- obtaining phased genotype information of a first parent and phased or unphased genotype information of a second parent;
- determining '(informative) loci', i.e. loci that are informative for the first parent using the genotype information of the first and second parent;
- categorizing continuous PVAF values of genetic material of the subject at said '(informative) loci' that are informative for the first parent in a category corresponding to the first parent;
- determining '(first subcategory) loci', i.e. loci where the first allele of the first parent comprises a genotype that is identical to the genotype of the two alleles of the second parent and subcategorizing continuous PVAF values at said '(first subcategory) loci' in a first subcategory;
- determining '(second subcategory) loci', i.e. loci where the second allele of the first parent comprises a genotype that is identical to the genotype of the two alleles of the second parent and subcategorizing continuous PVAF values at said '(second subcategory) loci' in a second subcategory;
- determining '(flipping) loci', i.e. loci where said first parent has a particular phased genotype;
- reflecting PVAF values at said determined '(flipping) loci' around the middle axis;
- segmenting said subcategorized PVAF values after the reflecting step; and
- providing the segmented PVAF values to indicate a genetic anomaly in the genetic material of the subject and/or inheritance of the genetic material of the subject.

The methods of the invention may comprise a categorizing and subcategorizing step. As is also evident from the wording of the embodiments (especially due to the use of the word comprising) the categorization and subcategorization steps do not need to be performed separately or in a particular order (the same applies to any of the method steps). In particular, the categorization and subcategorization may be performed simultaneously. As is evident from the description of the invention, preferably categorization and subcategorization are preformed based on the genotype information of the parents. Using the methods described herein, one can in a single step subcategorize PVAF values based on the (phased) genotype data of the parents.

For example, in another particular embodiment, the present invention provides a method comprising:

- obtaining continuous polymorphic variant allele frequency (PVAF) values of the genetic material of the subject;
- obtaining phased genotype information of a first parent and phased or unphased genotype information of a second parent;
- determining first subcategory loci where the first allele of the first parent comprises a genotype that is:

  - different from the genotype of the second allele of the first parent; and
  - identical to the genotype of the two (homozygous) alleles of the second parent;

- subcategorizing continuous PVAF values at said first subcategory loci in a first subcategory;
- determining second subcategory loci where the first allele of the first parent comprises a genotype that is:

  - different from the genotype of the second allele of the first parent; and
  - different to the genotype of the two (homozygous) alleles of the second parent;

- subcategorizing continuous PVAF values at said loci in a second subcategory;
- determining flipping loci where said first parent has a particular phased genotype;
- reflecting PVAF values in the first and second subcategory at determined flipping loci around the middle axis;
- segmenting said subcategorized PVAF values after the reflecting step; and
- providing the segmented PVAF values to indicate a genetic anomaly in the genetic material of the subject and/or inheritance of the genetic material of the subject.

In said instance, the subcategorization steps as described simultaneously categorize the PVAF values (i.e. retain PVAF values at loci informative for the first parent) and subcategorize the PVAF values (by distributing specific PVAF values at loci with specific allele combinations). Thus, based on the (phased) genotype, PVAF values may in a single step be assigned e.g. P1, P2, M1 and M2 subcategories.

It is further to be noted that the reflecting step may be performed before or during the categorization and/or subcategorization step. In particular, segmentation is performed after the reflecting step (if present in the method).

For example, the concept according to embodiments of the invention preferably comprises: phasing the parental PV-genotype(s), preferably using an available PV-genotype derived from a close relative, e.g. a sibling or the grandparents, or the cell-free, single-cell, few-cell or multi-cell DNA sample (either following WGA or not) genotype itself. In a preferred next step, the informative PV-loci are identified. In preferred embodiments, a locus is defined informative when in one parent it is heterozygous and in the other parent it is homozygous for the same PV-locus. In preferred embodiments informative PV-loci are categorized as informative parental categories, such as informative paternal or maternal. An informative locus is preferably defined as paternal when the father's PV-genotype at the locus is heterozygous and the mother's PV-genotype is homozygous. Similarly, an informative locus is defined as maternal when the mother's PV-genotype is heterozygous at the locus and the father's PV-genotype is homozygous. In further preferred embodiments, the maternal and paternal informative PV-loci, determined as described before, are further preferably subcategorized on the basis of specific parental informative PV-genotype combinations (herein also referred to as specific genotype combination of the first and second parent). Such that, in the paternal category, if the father's PV-genotype is AB and mother's PV-genotype is AA or if the father's PV-genotype is BA and the mother's PV-genotype is BB these loci are labeled as P1. And if the father's PV-genotype is AB and the mother's PV-genotype is BB or if the father's PV-genotype is BA and mother's PV-genotype is AA these are labeled as P2. Similarly in the maternal category, if the mother's PV-genotype is AB and the father's PV-genotype is AA or if the mother's PV-genotype is BA and the father's PV-genotype is BB these loci are labeled as M1. And if the mother's PV-genotype is AB and the father's PV-genotype is BB or if the mother's PV-genotype is BA and the father's PV-genotype is AA, these are labeled as M2.

It is to be noted that the subcategories P1, P2, M1 and M2 have the meaning as described in the present invention, which meaning does not necessarily corresponds to P1, P2, M1 or M2 described elsewhere (e.g. in EP1951897).

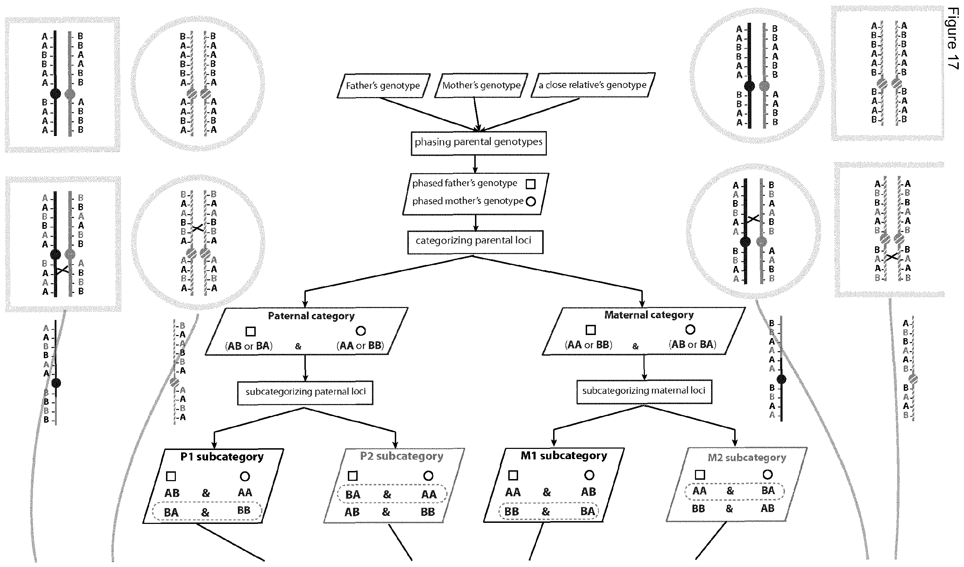

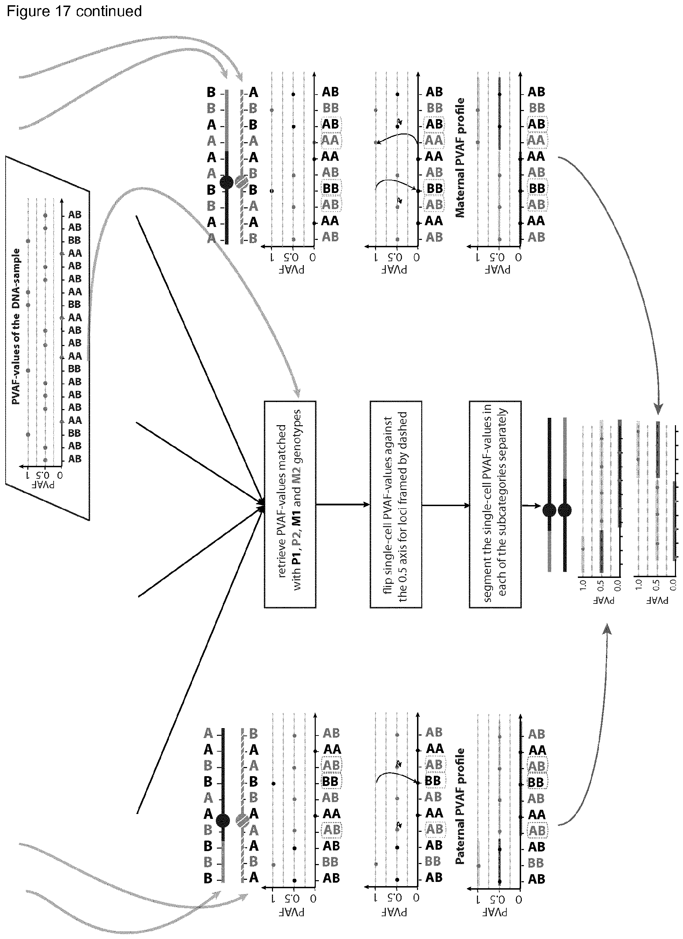

It is to be noted that, given the symmetry of a subject having two parents, embodiments describing the use of maternal genotype/haplotype information are applicable to the paternal equivalent as well, and vice-versa. For said reason, when references herein to first and second parent refer to father and mother or mother and father, respectively and vice-versa. Furthermore, the skilled person is aware on how to apply embodiments described using genotype/haplotype information of two parents to situations were only (phased) genotype/haplotype information of a single parent is used. As a mere example, if two phased genotypes are available (as is preferred and e.g. shown in fig. 17), PVAF values can be categorized in two categories (first parent and second parent) and each category can be further subcategorized in two subcategories based on the specific phased genotype combinations of the parents. In that situation, the methods of the invention allow to create a genetic profile of segmented PVAF values displaying genetic anomalies of the chromosomes inherited from both parents and indicating the inheritance/haplotype of the chromosomes inherited from both parents. However, such detailed information on chromosomes inherited from both parents is not always necessary. For example, if a father carries a genetic defect at a particular chromosomal region, identifying which paternal chromosome region has been inherited by the embryo/fetus/child allows to determine if the genetic defect has been transmitted. In addition, situations exist where (phased) genotype information is only available from a single parent. Therefore, the methods described herein may be applied using only genetic information of a single parent, or using only phased genotype information of a first parent and unphased genotype information of a second parent. E.g. if phased genotype information of the first parent is available, PVAF values can be categorized in the first parental category. Subcategorization can be based on the specific phased genotype of the first parent and unphased (homozygous) genotype of the second parent. Therefore, the methods of the present invention allow determining the genetic makeup of the chromosomes inherited from the first parent.

In a further embodiment PVAF data of cell-free, single-cell, few-cell or multi-cell derived DNA are categorized into two distinct paternal and maternal profiles according to the informative PV-loci determined as described above. Furthermore, in preferred embodiments the sample's PVAF data are reflected or mirrored around the middle axis when any of the parents have heterozygous BA PV-calls. More specifically, if the father has the BA genotype, matching cell-free, single-cell, few-cell or multi-cell DNA sample (either following WGA or not) PVAF values of the paternal profile are preferably reflected or mirrored around the middle axis. Similarly, if the mother has BA genotype, matching cell-free, single-cell, few-cell or multi-cell DNA sample (either following WGA or not) PVAF values of the maternal profile are reflected around the middle axis.

In preferred embodiments the sample's categorized PVAF data of the previous step are subcategorized, preferably into two sub-profiles per parental category (e.g. four subcategories in total analyzing the paternal and maternal categories) according to the informative loci determined before. More specifically, the sample's paternal PVAF data are preferably subcategorized into P1 and P2 sub-profiles. Similarly, the sample's maternal PVAF data are preferably subcategorized into M1 and M2 sub-profiles. Each of the four sub-profiles are preferably segmented, in a preferred embodiment using piecewise constant functions (PCF).

Beneficially over the prior art, the methods of the present invention allow for a high-quality analysis of genetic material using limited computing power. In particular, the methods of the invention can be applied using only PVAF values that have been categorized, while optionally discarding PVAF values which have not been categorized (e.g. PVAF values which do not correspond to informative loci). However, the methods of the invention use continuous PVAF values, which have a higher information content compared to discrete PV calls. The methods of the invention provide an optimal balance between reducing necessary data storage and computing power on the one hand (reduction to categorized PVAF values) and retaining a high information content (use of continuous PVAF values).

Optionally, preferred embodiments of the method provide that parental profiles can be visualized into two graphs. More specifically, by plotting P1 and P2 subprofiles in black and grey, respectively, generates the paternal profile graph; subsequently the segmented P1 and P2 are superimposed over P1 and P2 in the same graph. Similarly, plotting M1 and M2 sub-profiles in black and grey, respectively, generates maternal profile graph; subsequently the segmented M1 and M2 are superimposed over M1 and M2 in the same graph. The segmented P1, P2, M1 and M2 are used for further interpretation of the distinct signatures for different allelic architectures, as described below.

In another particular embodiment, the inherent parity feature (and/or complementarity feature) is used to assess the quality of the obtained segmented PVAF values, in particular to assess the segmentation quality. In further embodiment, the methods of the invention further comprise correcting the length of a segment in a first subcategory using the length of a corresponding segment in the second category. In another embodiment, the methods of the invention further comprise determining a quality score, wherein the quality score is determined by comparing segments of a first and second subcategory within a parental category. In a particular embodiment, comparing segments comprises comparing the length of segments. In another particular embodiment, comparing segments comprises comparing the location of segment ends. In a further embodiment, a quality score indicating a low quality is assigned if segments of a first and second subcategory are substantially different. A quality score indicating a high quality is assigned if segments of a first and second subcategory are substantially similar (i.e. the length and/or end of segments in two different subcategories are essentially the same or the difference is lower than a specified cutoff value). In another embodiment, providing the segmented PVAF values comprises evaluating the length of segments of segmented PVAF values in at least two subcategories of the category of a parent to indicate a homologous recombination site or a chromosomal breakpoint.

In another embodiment, the complementarity feature (and/or parity feature) is used to assess the quality of the obtained segmented PVAF values, in particular to assess the segmentation quality. In further embodiment, the methods of the invention further comprise correcting the distance between segments of different subcategories within a first category using the distance between segments of different subcategories within the second category. In particular correcting the paternal allelic distance ($d_{Pat}$) using the maternal allelic distance ($d_{Mat}$), or vice-versa. In another embodiment, the methods of the invention further comprise determining a quality score, wherein the quality score is determined by comparing the allelic distances in two parental categories. A quality score indicating a low quality is assigned if paternal and maternal allelic distances are substantially incompatible (not complementary). A quality score indicating a high quality is assigned if paternal and maternal allelic distances are substantially compatible (complementary) (i.e. the paternal and maternal allelic distances are essentially compatible). In a particular embodiment, the methods of the present invention (further) comprise determining the compatibility paternal allelic distances by comparing the sum of the allelic distances of the first and second parent to an expected value and/or a cutoff value. More in particular, the expected value of the sum of the paternal allelic distances is $PVAF_{max}$. E.g. if the continuous PVAF values are presented as numbers ranging from 0 to 1, the expected value is 1 (in particular $d_{Pat} + d_{Mat} = 1$). Thus, in a preferred embodiment, the sum of the paternal allelic distance and maternal allelic distance for a chromosome region is compared to 1. The sum being close to 1 indicates compatibility of the allelic distances. The sum being substantially different from 1 indicates incompatibility. Evidently, the sum of the paternal allelic distances at a chromosome region may be compared to a high and low cutoff value. Therefore, in a particular embodiment, if the sum of the parental allelic distances at a chromosome region is higher than the high cutoff value or lower than the low cutoff value, this indicates a low quality score. Similarly, the allelic distance in a particular parental category may be corrected using the allelic distance in the other parental category. More in particular, the allelic distance in a particular parental category may be corrected to 1 minus the allelic distance in the other parental category. E.g. $d_{Pat}(corr) = 1 - d_{Mat}$ or vice-versa. In another particular embodiment, providing the segmented PVAF values comprises for a chromosome region:

- determining a first distance between a) a segmented PVAF value in a first subcategory of the category of the first parent at said chromosome region and b) a segmented PVAF value in a second subcategory of the category of the first parent at said chromosome region;
- determining a second distance between a) a segmented PVAF value in a first subcategory of the category of the second parent at said chromosome region and b) a segmented PVAF value in a second subcategory of the category of the second parent at said chromosome region; and
- comparing the first distance and the second distance to indicate a copy number anomaly of said chromosome region.

In a particular embodiment, the present invention provides a report displaying the segmented PVAF values obtainable by the methods of the invention. In another embodiment, the present invention provides a report displaying segmented PVAF values of a chromosome region, wherein the segmented PVAF values have been subcategorized in at least two subcategories, wherein the distance between segments of different subcategories indicate the copy number of said chromosome region and wherein segment ends indicate a homologous recombination or chromosomal breakpoint in said chromosome region. Preferably, said report further displays the inheritance of said chromosome region. In another preferred embodiment, the present invention provides a haplotype structure (or "karyomap") of a chromosome or chromosome region, in particular of a whole chromosome, more in particular of two or more chromosomes. The haplotype structure (or karyomap) schematically represents said chromosome(s) or chromosome region(s) and indicates which regions have been inherited from which parental chromosome regions.

In a particular embodiment, the present invention provides a computer-assisted method for the analysis of genetic material of a subject, said method comprising:

- loading in a computer system continuous polymorphic variant allele frequency (PVAF) values of genetic material of a subject;
- loading in a computer system genotype information of a first and second parent; and
- by the computer system performing one or more of the remaining analysis steps of the method of the invention as described herein.

In a further particular embodiment, the present invention provides a computer-assisted method for the analysis of genetic material of a subject, said method comprising:

- loading in a computer system continuous polymorphic variant allele frequency (PVAF) values of genetic material of a subject;
- loading in a computer system genotype information of a first and second parent;
- categorizing by the computer system the continuous PVAF values in a category corresponding to the first parent based on the genotype information of the first parent;
- segmenting by the computer system said categorized PVAF values; and
- providing by the computer system the segmented PVAF values to indicate a genetic anomaly in the genetic material of the subject and/or inheritance of the genetic material of the subject.

In yet another particular embodiment, the present invention provides a computer-assisted method for the analysis of genetic material of a subject, said method comprising:

- loading in a computer system continuous polymorphic variant allele frequency (PVAF) values of genetic material of a subject;
- loading in a computer system phased genotype information of a first parent and phased or unphased genotype information of a second parent;
- categorizing by the computer system the continuous PVAF values in a category corresponding to the first parent based on the genotype information of the first and second parent;
- subcategorizing by the computer system the continuous PVAF values from the category corresponding to the first parent into subcategories;
- segmenting by the computer system said subcategorized PVAF values; and
- providing by the computer system the segmented PVAF values to indicate a genetic anomaly in the genetic material of the subject and/or inheritance of the genetic material of the subject.

In a particular embodiment, loading phased genotype information of a parent includes a) loading unphased genotype information of the parent, b) loading genotype information of a close relative, and c) phasing said unphased genotype data by a computer using the unphased genotype data of the parent and the genotype information of the close relative to obtain phased genotype information of the parent.

In yet another particular embodiment, the present invention provides a computer-assisted method for the analysis of genetic material of a subject, said method comprising:

- loading in a computer system continuous polymorphic variant allele frequency (PVAF) values of genetic material of a subject;
- loading in a computer system phased genotype information of a first parent and phased genotype information of a second parent;
- categorizing by the computer system the continuous PVAF values into a first category corresponding to the first parent and a second category corresponding to the second parent based on the genotype information of the first and second parent;
- subcategorizing by the computer system the continuous PVAF values in the first and second categories into subcategories;
- segmenting by the computer system said subcategorized PVAF values; and
- determining by the computer system the segmented PVAF values to indicate a genetic anomaly in the genetic material of the subject and/or inheritance of the genetic material of the subject.

In a further embodiment of the aforementioned computer-assisted methods of the present invention, the subcategorization of the PVAF values is combined with reflecting PVAF values at determined flipping loci around the middle axis as described herein.

*Cell-free, single-cell, few-cell or multi-cell DNA haplotyping for disomic chromosomes with balanced allelic architecture*

In preferred embodiments of the method, said method can comprise a multi-cell genotype of a sibling, named the reference sibling, which is serving as a seed for haplotyping. Hence, the multi-cell genotype of the reference sibling is preferably used to phase the multi-cell genotypes of the parents, assuming the parental homologous chromosomes transmitted to the reference sibling are P1 and M1. Applying the aforementioned principles of a method according to embodiments of the invention, on PVAF derived from cell-free, single-cell, few-cell or multi-cell DNA samples, paternal and maternal PVAF profiles can be generated. The segmented P1 and P2 PVAF-values, in the paternal profile of each chromosome reveal paternal haplotype blocks. Likewise, segmented M1 and M2 PVAF-values in the maternal profile of each chromosome reveal maternal haplotype blocks. The breakpoints in the segments of each PVAF-profile represent homologous recombination sites. In this embodiment, if in the paternal profile the segmented P1 and P2 PVAF-values are located at/around 0 and 0.5, respectively paternal alleles similar to the reference sibling are transmitted. However, if the segmented P1 and P2 PVAF-values are located at/around 0.5 and 1, respectively, paternal alleles distinct to the reference sibling are transmitted. Similarly, if in the maternal profile the segmented M1 and M2 PVAF-values are located at/around 0 and 0.5, respectively, maternal alleles similar to the reference sibling are transmitted. However, if the segmented M1 and M2 PVAF-values are located at/around 0.5 and 1, respectively, maternal alleles distinct to the reference sibling are transmitted (see e.g. Fig. 1). In other words, pairwise breakpoints in the segmented M1 and M2 single-cell SNP PVAF-values pinpoint maternal homologous recombination sites, while those in the segmented P1 and P2 single-cell PVAF-values locate paternal genetic crossovers. Hence, the resulting M1-M2 and P1-P2 segments denote inherited haplotype blocks from the phased parental genotypes.

In further embodiments of the invention, the method comprises the use of multi-cell PV-genotypes of the grandparents to phase the multi-cell PV-genotypes of the parents, assuming that the first parental homologous chromosome is from the grandfather and the second homologous chromosome is from the grandmother. Applying the aforementioned principles of the embodiments of the method according to the present invention on the PV B-allele frequencies, derived from cell-free, single-cell, few-cell or multi-cell DNA samples, paternal and maternal PVAF profiles of the sample are generated. The segmented P1 and P2 PVAF-values in the paternal PVAF-profile of each chromosome reveal paternal haplotype blocks. Likewise, segmented M1 and M2 PVAF-values in the maternal PVAF-profile of each chromosome reveal maternal haplotype blocks. In this embodiment, a pattern with segmented P1 and P2 PVAF-values located at/around 0 and 0.5, respectively, implies the inheritance of the paternal grandfather allele. However, a pattern with segmented P1 and P2 PVAF-values located at/around 0.5 and 1, respectively, implies the inheritance of the paternal grandmother allele. Similarly, a pattern with segmented M1 and M2 PVAF-values located at/around 0 and 0.5, respectively, implies the inheritance of the maternal grandfather allele. However, a pattern with segmented M1 and M2 PVAF-values located at/around 0.5 and 1, respectively, implies the inheritance of the maternal grandmother allele.

*Cell-free, single-cell, few-cell or multi-cell DNA haplotyping and copy-number profiling of genomic regions with imbalanced allelic architecture*

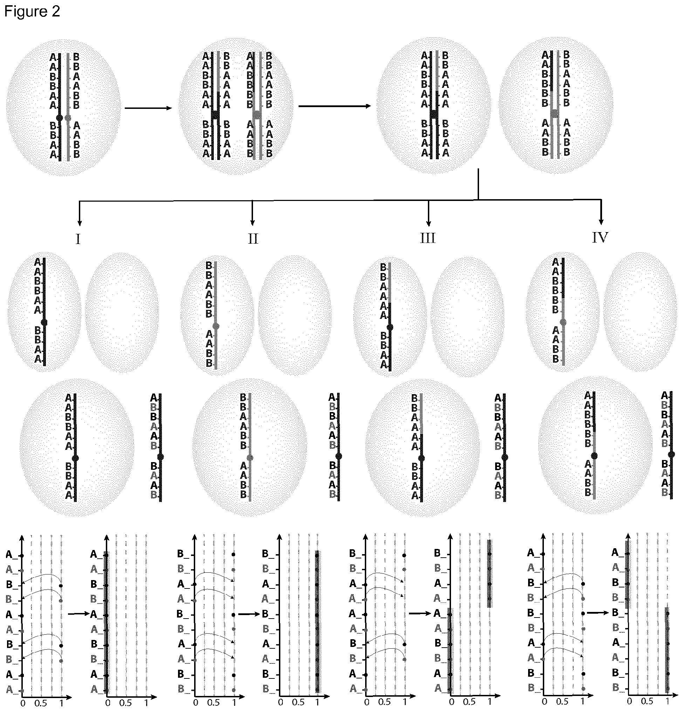

Allele/haplotype-specific copy number state as well as their parental and mechanistic origin can be determined using a method according to embodiments of the invention. The allelic imbalances in combination with matched (relative) DNA copy-number values (e.g. logR-values) result in further embodiments of the method.
In preferred embodiments of the method, the method provides that the ploidy state of a chromosome can be determined to be a monosomy with paternal origin, i.e. one paternal copy and no maternal copy, when the logR-values across that chromosome are around -1, but segmented P1 and P2 PVAF-values in the paternal PVAF-profile overlap and have values at/around either 0 or 1, while segmented M1 and M2 PVAF-values in the maternal PVAF-profile are at/around 0 and 1, respectively. In this embodiment, the breakpoints in the paternal PVAF-profile represent paternal homologous recombination sites. In this embodiment, if a reference sibling genotype is used for phasing the parental genotypes, a pattern with segmented and overlapping P1 and P2 PVAF-values at/around 0 implies the inheritance of the same paternal alleles as the reference sibling. However, a pattern with segmented and overlapping P1 and P2 PVAF-values at/around 1 implies the inheritance of the paternal alleles distinct to the reference sibling. In this embodiment, if the grandparental genotypes are used for phasing the parental genotypes, a pattern with segmented and overlapping P1 and P2 PVAF-values at/around 0 implies the inheritance of the paternal grandfather allele. However, a pattern with segmented and overlapping P1 and P2 PVAF-values at/around 1 implies the inheritance of the maternal grandmother allele. Figure 2 illustrates four likely paternal PVAF-profiles of a monosomy with a hypothetical homologous recombination event between the non-sister chromatids, assuming that the paternal allele is retained.

As can be seen from figure 2, prior to the step of reflecting PVAF values over the middle axis, PVAF values belonging to P1 and P2 categories lie around 0 and around 1. Segmenting said data would generate segments of P1 and P2 at 0 and segments of P1 and P2 at 1 in all four situations presented in figure 2. Therefore, e.g. the homologous recombination event shown in situations III and IV would not be visible from the segmented profiles. In particular, it is to be noted that figure 2 is a schematic representation of a hypothetical, "ideal" situations wherein PVAF values have the expected value of either 1 or 0. Furthermore, when using genotype data obtained from samples having a low amount of genetic material (such as single-cell samples), PVAF values will be noisy and the patterns before reflection and segmentation will be even less clear. The methods of the present invention allow for the first time to identify chromosomal breakpoints and/or homologues recombination sites in aneuploid samples, especially when said samples comprise low amounts of genetic material causing largely distorted and noisy PVAF values. These observations also apply for the other examples described herein.

In further embodiments of a method according to the present invention the ploidy state of a chromosome can be determined to be monosomy with maternal origin, i.e. no paternal copy and one maternal copy, when the logR-values across that chromosome are around -1, segmented M1 and M2 PVAF-values in the maternal PVAF-profile are overlapping and have values at/around either 0 or 1, and segmented P1 and P2 PVAF-values in the paternal PVAF-profile are apart and have values at/around 0 and 1, respectively. In this embodiment, the breakpoints in the maternal profile represent homologous recombination sites. In this embodiment, if a reference sibling genotype is used for phasing the parental genotypes, a pattern with segmented and overlapping M1 and M2 PVAF-values at/around 0 implies the inheritance of the same maternal alleles as the reference sibling. However, a pattern with segmented and overlapping M1 and M2 PVAF-values at/around 1 implies the inheritance of maternal alleles distinct to the reference sibling. In this embodiment, if the grandparental genotypes are used for phasing the parental genotypes, a pattern with segmented and overlapping M1 and M2 PVAF-values at/around 0 implies the inheritance of the maternal grandfather allele. However, a pattern with segmented and overlapping M1 and M2 PVAF-values at/around 1 implies the inheritance of the maternal grandmother allele. Figure 2 illustrates four likely maternal PVAF-profiles of a monosomy with a hypothetical homologous recombination event between the non-sister chromatids, assuming that the maternal allele is retained.

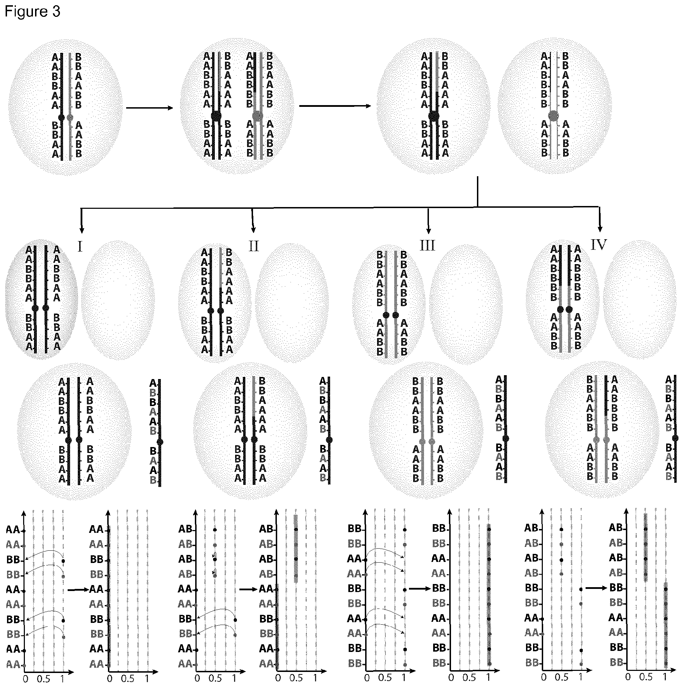

In yet further preferred embodiments, a chromosome can be determined to be uniparental isodisomy (UPiD) with paternal origin, i.e. two (similar) paternal copies and no maternal copy, when the logR-values across that chromosome are around 0, segmented P1 and P2 PVAF-values in the paternal profile are overlapping and have values at/around either 0 or 1, and segmented M1 and M2 PVAF-values in the maternal profile are apart and have values at/around 0 and 1, respectively, for same P1 and P2 PVAF-segments. In this embodiment, the breakpoints in the paternal profile represent homologous recombination sites. In this embodiment, if a reference sibling PV-genotype is used for phasing the parental genotypes, a pattern with segmented and overlapping P1 and P2 PVAF-values at/around 0 implies the inheritance of the same paternal alleles as the reference sibling. However, a pattern with segmented and overlapping P1 and P2 PVAF-values at/around 1 implies the inheritance of maternal alleles distinct to the reference sibling. In this embodiment, if the grandparental genotypes are used for phasing the parental genotypes, a pattern with segmented and overlapping P1 and P2 PVAF-values at/around 0 implies the inheritance of the paternal grandfather allele. However, a pattern with segmented and overlapping P1 and P2 PVAF-values at/around 1 implies the inheritance of the paternal grandmother allele. Figure 3 illustrates four most likely paternal PVAF-profiles of a chromosome with UPiD aberration with a hypothetical homologous recombination event between the non-sister chromatids, assuming that merely the paternal chromosomes are retained.

In preferred embodiments, a chromosome is determined to be uniparental isodisomy (UPiD) with maternal origin, i.e. two (similar) maternal copies and no paternal copy, when the logR-values across that chromosome are at/around 0, segmented M1 and M2 PVAF-values in the maternal PVAF-profile are overlapping and have values at/around either 0 or one, while segmented P1 and P2 PVAF-values in the paternal PVAF-profile are apart and have values at/around 0 and 1, respectively, for same M1 and M2 PVAF-segments. In this embodiment, the breakpoints in the maternal profile represent homologous recombination sites. In this embodiment, if a reference sibling genotype is used for phasing the parental genotypes, a pattern with segmented and overlapping M1 and M2 PVAF-values at/around 0 implies the inheritance of the same maternal alleles as the reference sibling. However, a pattern with segmented and overlapping M1 and M2 PVAF-values at/around 1 implies the inheritance of the maternal alleles distinct to the reference sibling. In this embodiment, if the grandparental genotypes are used for phasing of the parental genotypes, a pattern with segmented and overlapping M1 and M2 PVAF-values at/around 0 implies the inheritance of the maternal grandfather alleles. However, a pattern with segmented and overlapping M1 and M2 PVAF-values at/around 1 implies the inheritance of the maternal grandmother alleles. Figure 3 illustrates four most likely maternal PVAF-profiles of a chromosome with UPiD aberration with a hypothetical homologous recombination event between the non-sister chromatids, assuming that merely the maternal chromosomes are retained.

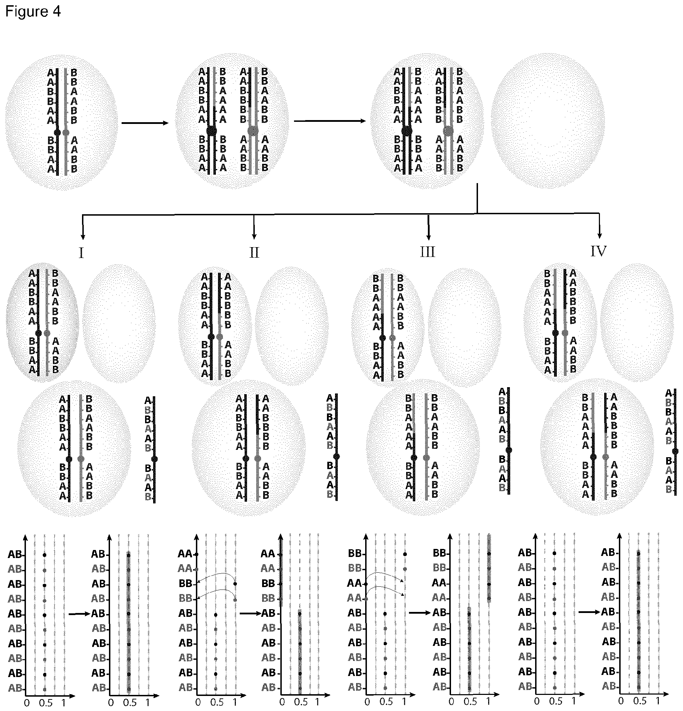

Embodiments of a method according to the invention provide that a chromosome can be determined to be uniparental heterodisomy (UPhD) with paternal origin, i.e. two (dissimilar) paternal copies (i.e. both paternal homologues) and no maternal copy, when the logR-values across that chromosome are at/around 0, segmented P1 and P2 PVAF-values in the paternal PVAF-profile are overlapping and are at/around 0.5 proximal to the centromere, and segmented P1 and P2 PVAF-values in the paternal profile are overlapping and are at/around 0 or 1 distal to the centromere if a homologous recombination site is present. In addition, segmented M1 and M2 PVAF-values in the maternal profile are apart and are at/around 0 and 1, respectively. Figure 4 illustrates four most likely paternal PVAF-profiles of a chromosome with UPhD aberration with a hypothetical homologous recombination event between the non-sister chromatids, assuming that merely the paternal chromosomes are retained.

Embodiments of a method according to the invention provide that a chromosome is determined to be uniparental heterodisomy (UPhD) with maternal origin, i.e. no paternal copy and two (dissimilar) maternal copies (i.e. both maternal homologues), when the logR-values across that chromosome are at/around 0, segmented M1 and M2 PVAF-values in the maternal profile are overlapping and are at/around 0.5 proximal to the centromere, and segmented M1 and M2 PVAF-values in the maternal profile are overlapping and have values at/around 0 or 1 distal to the centromere if a homologous recombination site is present. In addition, segmented P1 and P2 PVAF-values in the paternal PVAF-profile are apart and have values at/around 0 and 1, respectively. Figure 4 illustrates four most likely maternal PVAF-profiles of a chromosome with UPhD aberration with a hypothetical homologous recombination event between non-sister chromatids, assuming that merely the maternal chromosomes are retained.

Embodiments of a method according to the invention provide that the ploidy state of a chromosome can be determined to be trisomy of complete paternal origin, i.e. presence of three identical paternal chromosomes and no maternal copy, when the logR-values across that chromosome are around 0.58, segmented P1 and P2 PVAF-values in the paternal PVAF-profile are overlapping and are at/around either 0 or 1, and segmented M1 and M2 PVAF-values in the maternal PVAF-profile are apart at/around 0 and 1, respectively. In this embodiment, the breakpoints in the paternal profile represent homologous recombination sites. In this embodiment, if a reference sibling genotype is used for phasing the parental genotypes, a pattern with segmented and overlapping P1 and P2 PVAF-values at/around 0 implies the inheritance of the same paternal alleles as the reference sibling. And a pattern with segmented and overlapping P1 and P2 PVAF-values at/around 1 implies the inheritance of the paternal alleles distinct to those inherited by the reference sibling. In this embodiment, if the grandparental genotypes are used for phasing of the parental genotypes, a pattern with segmented and overlapping P1 and P2 PVAF-values at/around 0 implies the inheritance of the paternal grandfather allele. And a pattern with segmented and overlapping P1 and P2 PVAF-values at/around 1 implies the inheritance of the maternal grandmother allele. In this embodiment the final parental PVAF-profiles are similar to paternal PVAF-profiles of paternal monosomy or UPid PVAF-profiles (see e.g; Figs 2 and 3). However, the segmented logR-values are at/around 0.58.

Embodiments of a method according to the invention provide that the ploidy state of a chromosome can be determined to be trisomy with complete maternal origin, i.e. presence of three identical maternal copies and no paternal copy, when the logR-values across that chromosome are around 0.58, segmented M1 and M2 PVAF-values in the maternal PVAF-profile are overlapping and are at/around either 0 or 1, and segmented P1 and P2 PVAF-values in the paternal PVAF-profile are apart at/around 0 and 1, respectively. In this embodiment, the breakpoints in the maternal profile represent homologous recombination sites. In this embodiment, if a reference sibling genotype is used for phasing the parental genotypes, a pattern with segmented and overlapping M1 and M2 PVAF-values at/around 0 implies the inheritance of the same maternal alleles as the reference sibling. And a pattern with segmented and overlapping M1 and M2 PVAF-values at/around 1 implies the inheritance of the maternal alleles distinct to those inherited by the reference sibling. In this embodiment, if the grandparental genotypes are used for phasing the parental genotypes, a pattern with segmented and overlapping M1 and M2 PVAF-values at/around 0 implies the inheritance of the maternal grandfather allele. And a pattern with segmented and overlapping M1 and M2 PVAF-values at/around 1 implies the inheritance of the maternal grandmother allele. In this embodiment the final maternal PVAF-profiles are similar to maternal PVAF-profiles of maternal monosomy or UPid PVAF-profiles (see e.g. Figs 2 and 3), since only maternal copies are retained. However, the segmented logR-values are at/around 0.58.

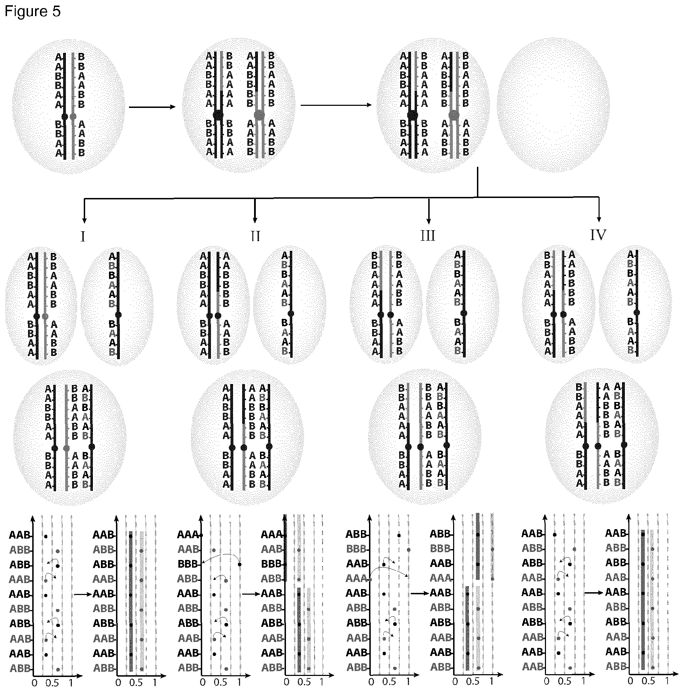

Embodiments of a method according to the invention provide that the ploidy state of a chromosome can be determined to be paternal trisomy, i.e. presence of two paternal copies and one maternal copy, with meiotic I origin when the logR-values across that chromosome are around 0.58, segmented P1 and P2 PVAF-values in the paternal profile are respectively around 0.33 and 0.67 proximal to the centromere and are around "0 and 0.33" or "0.67 and 1" distal to the centromere after a homologous recombination site. In this embodiment, segmented M1 and M2 PVAF-values in the maternal profile are respectively at/around 0 and 0.67 or at/around 0.33 and 1. In this embodiment, the breakpoints in the paternal and maternal PVAF-profiles represent homologous recombination sites. In this embodiment, if a reference sibling genotype is used for phasing the parental genotypes, a PVAF-pattern with segmented M1 and M2 PVAF-values at/around 0 and 0.67, respectively, implies the inheritance of the same maternal alleles as the reference sibling. And a pattern with segmented M1 and M2 PVAF-values at/around 0.33 and 1, respectively, implies the inheritance of the maternal alleles distinct to those inherited by the reference sibling. In this embodiment, if the grandparental genotypes are used for phasing the parental genotypes, a pattern with segmented M1 and M2 PVAF-values at/around 0 and 0.67 implies the inheritance of the maternal grandfather allele. And a pattern with segmented M1 and M2 PVAF-values at/around 0.33 and 1 implies the inheritance of the maternal grandmother allele. Figure 5 illustrates four most likely paternal PVAF-profiles of meiotic I trisomy with a hypothetical homologous recombination event between the non-sister chromatids, assuming that an extra paternal chromosome is transmitted.

Similar to the above comments regarding figure 2, without the reflection of certain PVAF values (based on the parental genotype), the recombination events in situations II and III can not be visualized. The present invention allows identifying the ploidy state (paternal trisomy) as well as the haplotype using a single method. Identifying the haplotype of aneuploid samples, even when such samples comprise low amounts of genetic material is an improvement over the prior art. This may, for example, be important in preimplantation diagnosis when the embryo has trisomy X. As limited to no phenotypic differences are associated with triple X, an embryo with trisomy X may still be implanted. Having the combined knowledge regarding the haplotype of the X chromosomes may provide valuable information regarding the transmission of a chromosome region carrying an X-linked genetic disorder from one of the parents to the embryo.

Embodiments of a method according to the invention provide that the ploidy state of a chromosome can be determined to be maternal trisomy, i.e. presence of two maternal copies and one paternal copy, with meiotic I origin when the logR-values across that chromosome are at/around 0.58, segmented M1 and M2 PVAF-values in the maternal PVAF-profile are respectively at/around 0.33 and 0.67 proximal to the centromere and are at/around "0 and 0.33" or "0.67 and 1" distal to the centromere after a homologous recombination site has occurred. In this embodiment, segmented P1 and P2 PVAF-values in the paternal profile are respectively at/around 0 and 0.67 or at/around 0.33 and 1. In this embodiment, the breakpoints in the paternal and maternal profiles represent homologous recombination sites. In this embodiment, if a reference sibling genotype is used for phasing the parental genotypes, a pattern with segmented P1 and P2 PVAF-values at/around 0 and 0.67, respectively, implies the inheritance of the same paternal alleles as the reference sibling. And a pattern with segmented P1 and P2 PVAF-values at/around 0.33 and 1, respectively, implies the inheritance of the paternal alleles distinct to those inherited by the reference sibling. In this embodiment, if the grandparental genotypes are used for phasing the parental genotypes, a pattern with segmented P1 and P2 PVAF-values at/around 0 and 0.67 implies the inheritance of the paternal grandfather allele.

And a pattern with segmented P1 and P2 PVAF-values at/around 0.33 and 1 implies the inheritance of the paternal grandmother allele. Figure 5 illustrates four most likely maternal PVAF-profiles of meiotic I trisomy with a hypothetical homologous recombination event between the non-sister chromatids, assuming that an extra maternal chromosome is transmitted.

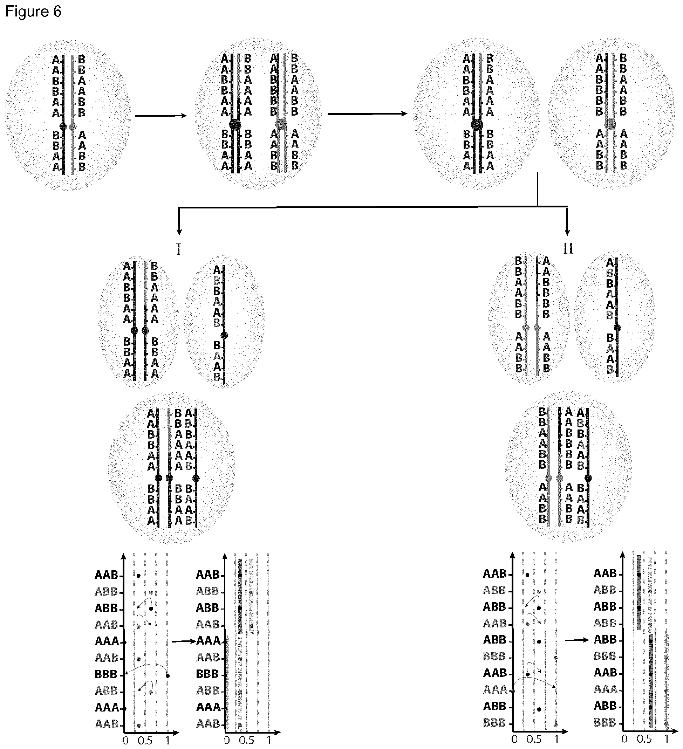

Embodiments of a method according to the invention provide that the ploidy state of a chromosome can be determined to be paternal trisomy, i.e. presence of two paternal copies and one maternal copy, with meiotic II origin when the logR-values across that chromosome are around 0.58, segmented P1 and P2 PVAF-values in the paternal profile are at/around 0 and 0.33 or at/around 0.67 and 1 proximal to the centromere and are at/around 0.33 and 0.67 distal to the centromere after a homologous recombination site is present. In this embodiment, segmented M1 and M2 PVAF-values in the maternal profile are respectively around 0 and 0.67 or at/around 0.33 and 1. In this embodiment, the breakpoints in the paternal and maternal profiles represent homologous recombination sites. In this embodiment, if a reference sibling genotype is used for phasing the parental genotypes, a pattern with segmented M1 and M2 PVAF-values at/around 0 and 0.67, respectively, implies the inheritance of the same maternal alleles as the reference sibling. However, a pattern with segmented M1 and M2 PVAF-values at/around 0.33 and 1, respectively, implies the inheritance of the maternal alleles distinct to those inherited by the reference sibling. In this embodiment, if the grandparental genotypes are used for phasing the parental genotypes, a pattern with segmented M1 and M2 PVAF-values at/around 0 and 0.67 implies the inheritance of the maternal grandfather. However, a pattern with segmented M1 and M2 PVAF-values at/around 0.33 and 1 implies the inheritance of the maternal grandmother. Figure 6 illustrates two most likely paternal PVAF-profiles of meiotic II trisomy with a hypothetical homologous recombination event between the non-sister chromatids, assuming that an extra paternal chromosome is transmitted.

Embodiments of a method according to the invention provide that the ploidy state of a chromosome can be determined to be maternal trisomy, i.e. presence of one paternal copy and two maternal copies, with meiotic II origin when the logR-values across that chromosome are at/around 0.58, segmented M1 and M2 PVAF-values in the maternal profile are at/around 0 and 0.33 or at/around 0.67 and 1 proximal to the centromere and are at/around 0.33 and 0.67 distal to the centromere after a homologous recombination site has occurred. In this embodiment, segmented P1 and P2 PVAF-values in the paternal profile are respectively around 0 and 0.67 or at/around 0.33 and 1. In this embodiment, the breakpoints in the paternal and maternal profiles represent homologous recombination sites. In this embodiment, if a reference sibling genotype is used for phasing the parental genotypes, a pattern with segmented P1 and P2 PVAF-values at/around 0 and 0.67, respectively, implies the inheritance of the same paternal alleles as the reference sibling. However, a pattern with segmented and P1 and P2 PVAF-values at/around 0.33 and 1, respectively, implies the inheritance of the paternal alleles distinct to those inherited by the reference sibling. In this embodiment, if the grandparental genotypes are used for phasing the parental genotypes, a pattern with segmented P1 and P2 PVAF-values at/around 0 and 0.67 implies the inheritance of the paternal grandfather allele. However, a pattern with segmented P1 and P2 PVAF-values at/around 0.33 and 1 implies the inheritance of the paternal grandmother allele. Figure 6 illustrates two most likely maternal PVAF-profiles of meiotic II trisomy with a hypothetical homologous recombination event between the non-sister chromatids, assuming that an extra maternal chromosome is transmitted.

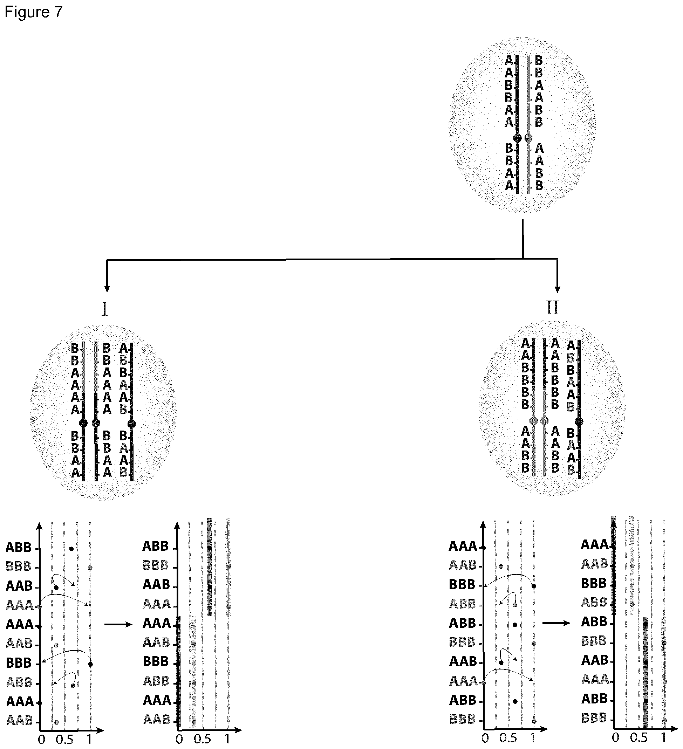

Embodiments of a method according to the invention provide that the ploidy state of a chromosome can be determined to be paternal trisomy, i.e. presence of two paternal copies and one maternal copy, with mitotic origin when the logR-values across that chromosome are around 0.58, segmented P1 and P2 PVAF-values in the paternal profile are at/around 0 and 0.33 or at/around 0.67 and 1, respectively. In this embodiment, segmented M1 and M2 PVAF-values in the maternal profile are respectively at/around 0 and 0.67 or at/around 0.33 and 1. In this embodiment, the breakpoints in the paternal and maternal profiles represent homologous recombination sites. In this embodiment, if a reference sibling genotype is used for phasing of the parental genotypes, a pattern with segmented P1 and P2 PVAF-values at/around 0 and 0.33, respectively, implies the inheritance and duplication of the same paternal alleles as those inherited by the reference sibling. However, a pattern with segmented P1 and P2 PVAF-values around 0.67 and 1, respectively, implies the inheritance and duplication of the paternal alleles distinct to those inherited by the reference sibling. In addition, in the maternal profile a pattern with segmented M1 and M2 PVAF-values at/around 0 and 0.67, respectively, implies the inheritance of the same maternal alleles as the reference sibling. However, a pattern with segmented M1 and M2 PVAF-values at/around 0.33 and 1, respectively, implies the inheritance of the maternal alleles distinct to those inherited by the reference sibling. In this embodiment, if the grandparental genotypes are used for phasing the parental genotypes, a pattern with segmented P1 and P2 PVAF-values around 0 and 0.33 implies the inheritance of maternal grandfather alleles. However, a pattern with segmented P1 and P2 PVAF-values around 0.67 and 1 implies the inheritance of paternal grandmother alleles. In addition, in the maternal profile a pattern with segmented M1 and M2 PVAF-values around 0 and 0.67 implies the inheritance of maternal grandfather alleles. However, a pattern with segmented M1 and M2 PVAF-values around 0.33 and 1 implies the inheritance of maternal grandmother alleles. Figure 7 illustrates two most likely paternal PVAF-profiles of mitotic trisomy with a hypothetical homologous recombination event between the non-sister chromatids, assuming that the paternal chromosome is duplicated.

Embodiments of a method according to the invention provide that the ploidy state of a chromosome can be determined to be maternal trisomy, i.e. presence of one paternal copy and two maternal copies, with mitotic origin when the logR-values across that chromosome are at/around 0.58, segmented M1 and M2 PVAF-values in the maternal PVAF-profile are around 0 and 0.33 or around 0.67 and 1, respectively. In this embodiment, segmented P1 and P2 PVAF-values in the paternal PVAF-profile are respectively at/around 0 and 0.67 or at/around 0.33 and 1. In this embodiment, the breakpoints in the paternal and maternal PVAF-profiles represent homologous recombination sites. In this embodiment, if a reference sibling genotype is used for phasing the parental genotypes, a pattern with segmented M1 and M2 PVAF-values at/around 0 and 0.33, respectively, implies the inheritance and duplication of the same maternal alleles as carried by the reference sibling. However, a pattern with segmented M1 and M2 PVAF-values at/around 0.67 and 1, respectively, implies the inheritance of the maternal alleles distinct to those inherited by the reference sibling. In addition, in the paternal PVAF-profile a pattern with segmented P1 and P2 PVAF-values at/around 0 and 0.67, respectively, implies the inheritance of the same paternal alleles as the reference sibling. However, a pattern with segmented P1 and P2 PVAF-values at/around 0.33 and 1, respectively, implies the inheritance of the paternal alleles distinct to those inherited by the reference sibling. In this embodiment, if the grandparental genotypes are used for phasing the parental genotypes, a pattern with segmented M1 and M2 PVAF-values around 0 and 0.33 implies the inheritance of maternal grandfather alleles. However, a pattern with segmented M1 and M2 PVAF-values around 0.67 and 1 implies the inheritance of maternal grandmother alleles. In addition, in the paternal profile a pattern with segmented P1 and P2 PVAF-values around 0 and 0.67 implies the inheritance of paternal grandfather alleles. However, a pattern with segmented P1 and P2 PVAF-values around 0.33 and 1 implies the inheritance of paternal grandmother alleles. Figure 7 illustrates two most likely maternal PVAF-profiles of mitotic trisomy with a hypothetical homologous recombination event between the non-sister chromatids, assuming that the maternal chromosome is duplicated.

Embodiments of a method according to the invention provide that the ploidy state of a chromosome can be determined to be tetrasomy, i.e. presence of two paternal copies and two maternal copies, when the logR-values across that chromosome are around 1. In this embodiment, the segmented P1 and P2 PVAF-values in the paternal profile are respectively at/around 0 and 0.5 or at/around 0.5 and 1. Likewise, the segmented M1 and M2 PVAF-values in the maternal profile are respectively at/around 0 and 0.5 or at/around 0.5 and 1. In this embodiment, the breakpoints in the paternal and maternal profiles represent homologous recombination sites. In this embodiment, if a reference sibling genotype is used for phasing the parental genotypes, a pattern with segmented P1 and P2 PVAF-values at/around 0 and 0.5, respectively, implies the inheritance of the same paternal alleles as carried by the reference sibling. And a pattern with segmented P1 and P2 PVAF-values at/around 0.5 and 1, respectively, implies the inheritance of the paternal alleles distinct to those inherited by the reference sibling. Likewise, a pattern with segmented M1 and M2 PVAF-values around 0 and 0.5, respectively, implies the inheritance of the same maternal alleles as carried by the reference sibling. And a pattern with segmented M1 and M2 PVAF-values around 0.5 and 1, respectively, implies the inheritance of the maternal alleles distinct to those inherited by the reference sibling. In this embodiment, if the grandparental genotypes are used for phasing the parental genotypes, a pattern with segmented P1 and P2 PVAF-values around 0 and 0.5, respectively, implies the inheritance of the paternal grandfather allele. And a pattern with segmented P1 and P2 PVAF-values around 0.5 and 1, respectively, implies the inheritance of the paternal grandmother allele. Likewise, a pattern with segmented M1 and M2 PVAF-values around 0 and 0.5, respectively, implies the inheritance of the maternal grandfather allele. And a pattern with segmented M1 and M2 PVAF-values around 0.5 and 1, respectively, implies the inheritance of the maternal grandmother allele. In this embodiment the final parental PVAF-profiles are similar to parental PVAF-profiles of a normal disomic chromosome. However, the segmented logR-values are at/around 1.

In some embodiments, the ploidy state and its origin can be determined by using merely trio data, i.e. parents and an offspring. In this embodiment the genotype of the offspring used to phased parental genotypes. Applying the aforementioned principles (ii to ix) on the PV B-allele frequencies derived from cell-free, single-cell, few-cell or multi-cell DNA of the embryo/foetus/offspring/tumor can reveal the parental and mechanistic origin of a chromosomal copy number or copy neutral aberration.

*Multi-cell haplotype and copy-number profiling for detecting mosaic architecture in bulk of cells*

Embodiments of a method according to the invention provide that a chromosome can be determined to be mosaic monosomy with maternal origin. In this embodiment a bulk of cells may comprise for instance two cell populations: (1) a p% abnormal cells, i.e. cells with one maternal copy and no paternal copy, and (2) (100 - p)% normal cells, i.e. cells with one maternal copy and one paternal copy. In this embodiment the logR-values across that chromosome are between 0 to -1 (depending on the degree of mosaicism p%), segmented M1 and M2 PVAF-values in the maternal PVAF-profile are approaching each other and have an overall distance of <0.5, whereas segmented P1 and P2 PVAF-values in the paternal PVAF-profile are repelling each other and have an overall distance of >0.5, for the same M1 and M2 PVAF-segments. In this embodiment the distance between M1 and M2 and between P1 and P2 as well as the segmented logR-values reflect the degree of mosaicism p%. In this embodiment, the breakpoints preferably represent homologous recombination sites.

Embodiments of a method according to the invention provide that a chromosome can be determined to be mosaic monosomy with paternal origin. In this embodiment a bulk of cells may comprise for instance of two cell populations: (1) a p% abnormal cells, i.e. cells with one paternal copy and no maternal copy, and (2) (100 - p)% normal cells, i.e. cells with one maternal copy and one paternal copy. In this embodiment the logR-values across that chromosome are between 0 to -1 (depending on the degree of mosaicism p%), segmented P1 and P2 PVAF-values in the maternal PVAF-profile are approaching each other and thus have an overall distance of <0.5, whereas segmented M1 and M2 PVAF-values in the maternal PVAF-profile are repelling each other and thus have an overall distance of >0.5, for the same P1 and P2 PVAF-segments. In this embodiment the distance between M1 and M2 and between P1 and P2, as well as the segmented logR-values reflect the degree of mosaicism $\rho$%. In this embodiment, the breakpoints represent homologous recombination sites.

Embodiments of a method according to the invention provide that a chromosome can be determined to be mosaic uniparental disomy with maternal origin. In this embodiment a bulk of cells may comprise for instance of two cell populations: (1) a p% abnormal UPD cells, i.e. cells with two identical maternal copies and no paternal copy, and (2) (100 - p)% normal cells, i.e. cells with one maternal copy and one paternal copy. In this embodiment the logR-values across that chromosome are at/around 0, segmented M1 and M2 PVAF-values in the maternal PVAF-profile are approaching each other and have an overall distance of <0.5, whereas segmented P1 and P2 PVAF-values in the paternal PVAF-profile are repelling each other and have an overall distance of >0.5, for the same M1 and M2 PVAF-segments. In this embodiment the distance between M1 and M2 and the distance between P1 and P2 reflect the degree of mosaicism $\rho$%. In this embodiment, the breakpoints represent homologous recombination sites. Figure 8 illustrates a mosaic UPD with $\rho$=50% degree of mosaicism.

Embodiments of a method according to the invention provide that a chromosome can be determined to be mosaic uniparental disomy with paternal origin. In this embodiment a bulk of cells may comprise of two populations: (1) a $\rho$% abnormal UPD cells, i.e. cells with two identical paternal copies and no maternal copy, and (2) (100 - $\rho$)% normal cells, i.e. cells with one maternal copy and one paternal copy. In this embodiment the logR-values across that chromosome are at/around 0, segmented P1 and P2 PVAF-values in the paternal PVAF-profile are approaching each other and have an overall distance of <0.5, whereas segmented M1 and M2 PVAF-values in the maternal PVAF-profile are repelling each other and have an overall distance of >0.5, for the same M1 and M2 PVAF-segments. In this embodiment the distance between M1 and M2 and the distance between P1 and P2 reflect the degree of mosaicism ρ%. In this embodiment, the breakpoints represent homologous recombination sites. Figure 8 illustrates a mosaic UPD with ρ=50% degree of mosaicism.

Embodiments of a method according to the invention provide that a chromosome can be determined to be mosaic trisomy with maternal origin. In this embodiment a bulk of cells may comprise for instance of two populations: (1) a ρ% abnormal trisomy cells, i.e. cells with two maternal copies and one paternal copy, and (2) (100 - ρ)% normal cells, i.e. cells with one maternal copy and one paternal copy. In this embodiment the logR-values across that chromosome are between 0 and 0.58, segmented M1 and M2 PVAF-values in the maternal PVAF-profile are approaching each other and have an overall distance of <0.5, whereas segmented P1 and P2 PVAF-values in the paternal PVAF-profile are repelling each other and have an overall distance of >0.5, for the same M1 and M2 PVAF-segments. In this embodiment the segmented logR-values, the distance between M1 and M2 and the distance between P1 and P2 reflect the degree of mosaicism ρ%. In this embodiment, the breakpoints represent homologous recombination sites.

Embodiments of a method according to the invention provide that a chromosome can be determined to be mosaic trisomy with paternal origin. In this embodiment a bulk of cells may comprise for instance of two populations: (1) a ρ% abnormal trisomy cells, i.e. cells with two paternal copies and one maternal copy, and (2) (100 - ρ)% normal cells, i.e. cells with one maternal copy and one paternal copy. In this embodiment the logR-values across that chromosome are between 0 and 0.58, segmented P1 and P2 PVAF-values in the paternal PVAF-profile are approaching each other and have an overall distance of <0.5, whereas segmented M1 and M2 PVAF-values in the maternal PVAF-profile are repelling each other and have an overall distance of > 0.5, for the same P1 and P2 PVAF-segments. In this embodiment the segmented logR-values, the distance between M1 and M2 and the distance between P1 and P2 reflect the degree of mosaicism ρ%. In this embodiment, the breakpoints represent homologous recombination sites.

## Optional normalization procedure

In further preferred embodiments, where DNA-samples derived from cells with chromosome instability are used, such as e.g. DNA-samples derived from human cleavage-stage embryos or cancer cells, (relative) DNA copy-number values (e.g. logR-values) are preferably normalized for proper copy-number analysis. In this embodiment the normalization process preferably comprises determination of the most likely normal disomic chromosomes for logR-value correction. Allele-specific parent-of-origin values may be determined as described by Voet et al in "Breakage-fusion-bridge cycles leading to inv dup del occur in human cleavage stage embryos", Human mutation 32, 783 (2011). Following chromosome-specific parental score calculation, parental relative ratios are computed and used for preliminary normalization of the (relative) DNA copy-number values (e.g. logR-values). In this embodiment, paternal and maternal scores, $PS_k$ and $MS_k$, respectively, can be computed for each chromosome k, given allele specific parent-of-origin values:

$$PS_k = \frac{\sum_j P_{k,j}}{\sum_j S_{k,j}}$$

$$MS_k = \frac{\sum_j M_{k,j}}{\sum_j S_{k,j}}$$

where $P_{k,j}$ and $M_{k,j}$ represent paternal and maternal parent-of-origin values for locus j on chromosome k, respectively; and $S_{k,j}$ is a PV-call on chromosome k that is informative for parent-of-origin analysis, including the parental PV-calls that are not both heterozygous or identical homozygous. In this embodiment, subsequent parental relative ratio computation, may comprise:

$$Pat_k = \frac{PS_k}{PS_k + MS_k}$$

$$Mat_k = \frac{MS_k}{PS_k + MS_k}$$

where $Pat_k$ and $Mat_k$ are the paternal and maternal relative ratios, respectively, of the chromosome k. In preferred embodiment, raw (relative) DNA copy-number values (e.g. logR-values) are smoothed using a sliding window. Said smoothed/raw (relative) DNA copy-number values (e.g. logR-values) are preferably corrected for %GC-content bias by a loess-fit and the (relative) DNA copy-number values (e.g. logR-values) are preliminary normalized using a (trimmed) mean of the likely normal disomic chromosomes determined by aforementioned parental scoring criteria. In this embodiment, integrating said preliminary (relative) DNA copy-number value (e.g. logR-values) profiles with haplotyped / phased and segmented PVAF-values, a final selection of chromosomes is made and used for consequent (trimmed) mean correction of said preliminary (relative) DNA copy-number values (e.g. logR-values). In addition, normalized (relative) DNA copy-number values (e.g. logR-values) which are subsequently integrated with haplotyped / phased and segmented single-cell PVAF values can be used to call DNA-aberrations. For instance for nullisomic, monosomic, disomic, uniparental disomic and trisomic loci typical patterns in haplotyped / phased and segmented PVAF-values can be expected.

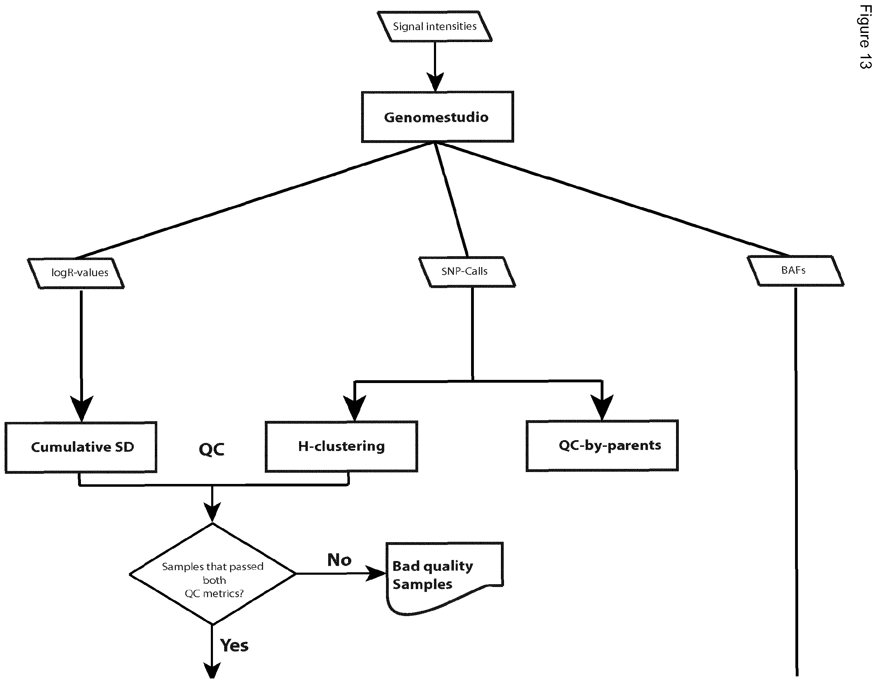

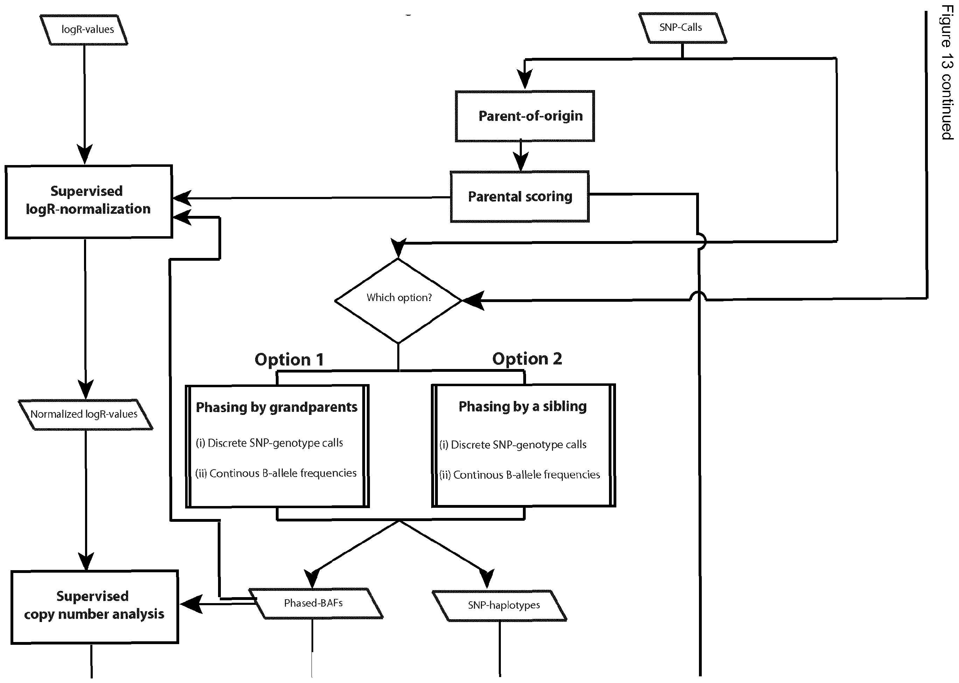

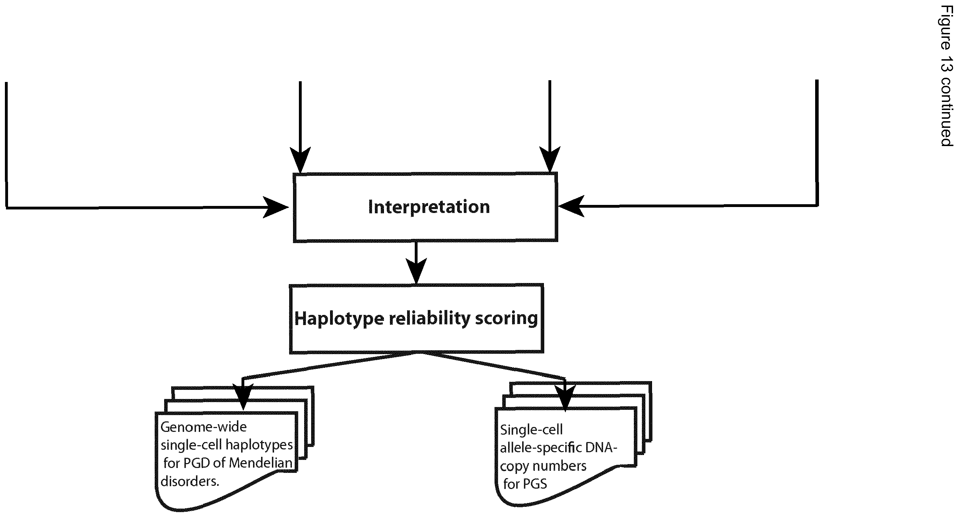

Figs. 13a-13c illustrate a flowchart of the computational pipeline, comprising a module based on a method according to embodiments of the invention. Said computational pipeline advantageously provides single-cell haplotyping and imputation of linked disease variants. Besides the modules for QC-ing, genotyping, bimodal haplotyping and visualizing the SNP-array data, the module comprising a method according to embodiments of the invention, can further be equipped with a supervised copy number analysis module allowing not only the identification of chromosomal imbalances and their parental origin, but also the detection of copy neutral DNA-aberrations as uniparental disomies (UPDs). This module in addition may allow discovering the meiotic or mitotic nature of chromosomal anomalies.

*Optional PVAF-value transformation*

In further optional embodiments, continuous PVAF-values in the paternal and maternal categories can be transformed to discrete paternal and maternal haplotypes.

In this embodiment, due to the parity and complementarity features (see definitions) of the present invention, the paternal haplotype of the DNA-sample using all genetic PVAF-values of the entire genome or a selection thereof can be reconstructed. Whereby said transformation may comprise:

    i. Iteratively computing the distance between each raw P1 PVAF-value with the nearest neighbor P2 PVAF-value;
    ii. Assigning the computed distances to the corresponding P1 loci;
    iii. Adding the assigned distances to the corresponding raw P1-values, concatenate the latter to the P2 and sort the entiere array based on their physical position, the resulting values are called adjusted P1 (adj-P1);
    iv. Iteratively computing the distance between each raw P2 PVAF-value with the nearest neighbor P1 PVAF-value;
    v. Assigning the computed distances to the corresponding P2 loci;
    vi. Substracting the assigned distances from the corresponding raw P2-values, concatenate the latter to the P1 and sort the entiere array based on their physical position, the resulting values are called adjusted P2 (adj-P2);
    vii. Omitting all the values less than a certain threshold (e.g. 0.8) from the from the resulting adj-P1 and adj-P2;
    viii. Omitting all the values greater than a certain threshold (e.g. 0.2) from the resulting adj-P1 and adj-P2;
    ix. Reassigning all the remaining values less than 0.5 in the paternal category to 1 (i.e. same haplotype block as the close relative is transmitted);
    x. Reassigning all the remaining values greater than 0.5 in the paternal category to 2 (i.e. distinct haplotype block as the close relative is transmitted);

In this embodiment, due to parity and complementarity features of the present invention, the maternal haplotype of the DNA-sample using all genetic PVAF-values of the entire genome or a selection thereof can be reconstructed. Whereby said transformation may comprise:

    i. Iteratively computing the distance between each raw M1 PVAF-value with the nearest neighbor M2 PVAF-value;
    ii. Assigning the computed distances to the corresponding M1 loci;
    iii. Adding the assigned distances to the corresponding raw M1-values, concatenate the latter to the M2 and sort the entiere array based on their physical position, the resulting values are called adjusted M1 (adj-M1);
    iv. Iteratively computing the distance between each raw M2 PVAF-value with the nearest neighbor M1 PVAF-value;
    v. Assigning the computed distances to the corresponding M2 loci;
    vi. Substracting the assigned distances from the corresponding raw M2-values, concatenate the latter to the M1 and sort the entiere array based on their physical position, the resulting values are called adjusted M2 (adj-M2);
    vii. Omitting all the values less than a certain threshold (e.g. 0.8) from the resulting adj-M1 and adj-M2;
    viii. Omitting all the values greater than a certain threshold (e.g. 0.2) from the resulting adj-M1 and adj-M2;
    ix. Reassigning all the remaining values less than 0.5 in the maternal category to 1 (i.e. same haplotype block as the close relative is transmitted);
    x. Reassigning all the remaining values greater than 0.5 in the maternal category to 2 (i.e. distinct haplotype block as the close relative is transmitted);

Figure 15 illustrates the accuracy of the present invention on homologous recombination site (HR-site) detection.

It is to be understood that this invention is not limited to the particular features of the means and/or the process steps of the methods described as such means and methods may vary. It is also to be understood that the terminology used herein is for purposes of describing particular embodiments only, and is not intended to be limiting. It must be noted that, as used in the specification and the appended claims, the singular forms "a" "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

## Claims

1. A computer-implemented method for the analysis of genetic material of a subject, said method comprising:

   - obtaining continuous polymorphic variant allele frequency (PVAF) values of the genetic material of the subject;
   - obtaining phased genotype information of a first parent and phased or unphased genotype information of a second parent;
   - categorizing the continuous PVAF values in a first category corresponding to the first parent based on the genotype information of the first and second parent;
   - subcategorizing the continuous PVAF values from the first category corresponding to the first parent into subcategories;
   - segmenting said subcategorized PVAF values; and
   - providing the segmented PVAF values to indicate a genetic anomaly in the genetic material of the subject and/or inheritance of the genetic material of the subject.

2. The method of claim 1, wherein the genotype information of the second parent is phased genotype information;
   further comprising

   - categorizing the continuous PVAF values into a second category corresponding to the second parent based on the genotype information of the first and second parent; and
   - subcategorizing the continuous PVAF values in the first and second categories into subcategories.

3. The method of any one of the previous claims, further comprising reflecting obtained PVAF values against the middle axis prior to segmentation.

4. The method of claim 1 or 2, further comprising reflecting the obtained PVAF values, at a position corresponding to a specific phased parental genotype, against the middle axis prior to segmentation.

5. The method of any one of the previous claims, further comprising obtaining DNA quantity values, such as logR values or read count values, and normalizing said DNA quantity values based on said segmented PVAF values.

6. The method of any one of the previous claims, wherein the continuous PVAF values have been determined using a sample comprising a low amount of genetic material of said subject, such as a sample comprising only one or a few cells of said subject, or a plasma sample obtained from a mother pregnant with said subject.

7. The method of any one of the previous claims, wherein the genetic anomaly indicated by the segmented PVAF values comprises a numerical or structural chromosomal abnormality, or mosaicism.

8. The method of any one of the previous claims, wherein the segmented PVAF values indicate the meiotic or mitotic origin of a genetic anomaly in the genetic material of the subject.

9. The method of any one of the previous claims, wherein the segmented PVAF values indicate the haplotype of the genetic material of the subject.

10. The method of any one of the previous claims, wherein the segmented PVAF values indicate the copy number of a chromosome or chromosome region in the genetic material of the subject and/or a homologous recombination site or a chromosomal breakpoint.

11. The method of claim 1 or 2, wherein providing the segmented PVAF values comprises evaluating the length of segments of segmented PVAF values in at least two subcategories of the category of a parent to indicate a homologous recombination site or a chromosomal breakpoint.

12. The method of claim 2, wherein providing the segmented PVAF values comprises for a chromosome region:

    - determining a first distance between a) a segmented PVAF value in a first subcategory of the category of the first parent at said chromosome region and b) a segmented PVAF value in a second subcategory of the category of the first parent at said chromosome region;
    - determining a second distance between a) a segmented PVAF value in a first subcategory of the category of the second parent at said chromosome region and b) a segmented PVAF value in a second subcategory of the category of the second parent at said chromosome region; and
    - comparing the first distance and the second distance to indicate a copy number anomaly of said chromosome region.

**13.** The method of any one of the previous claims, wherein categorizing the continuous PVAF values in a category corresponding to the first parent comprises:

- determining loci that are informative for the first parent using the genotype information of the first and second parent; and
- categorizing continuous PVAF values of genetic material of the subject at said loci that are informative for the first parent in a category corresponding to the first parent.

**14.** The method of claim 1, wherein subcategorizing the continuous PVAF values from the category corresponding to the first parent comprises:

- determining loci with a specific genotype combination of the first and second parent;
- subcategorizing continuous PVAF values of genetic material of the subject at said loci with a specific genotype combination of the first and second parent.

**15.** A computer program product which is capable, when executed on a processing engine, to perform the method of any one of the previous claims.

**16.** A non-transitory machine-readable storage medium storing the computer program product of claim 15.

**Patentansprüche**

**1.** Ein computerimplementiertes Verfahren zur Analyse von genetischem Material einer Testperson, wobei das erwähnte Verfahren Folgendes umfasst:

- Erhalten kontinuierlicher Werte von Allelfrequenzen mit polymorphen Varianten (PVAF-Werte) des genetischen Materials der Testperson;
- Erhalten phasierter Genotypinformation von einem ersten Elternteil und phasierter oder unphasierter Genotypinformation von einem zweiten Elternteil;
- Kategorisieren der kontinuierlichen PVAF-Werte in eine erste Kategorie, welche dem ersten Elternteil entspricht, basierend auf der Genotypinformation vom ersten und zweiten Elternteil;
- Subkategorisieren der kontinuierlichen PVAF-Werte aus der ersten Kategorie entsprechend dem ersten Elternteil in Subkategorien;
- Segmentieren der erwähnten subkategorisierten PVAF-Werte; und
- Bereitstellen der segmentierten PVAF-Werte, um eine genetische Anomalie im genetischen Material der Testperson und/oder Vererbung des genetischen Materials der Testperson kenntlich zu machen.

**2.** Das Verfahren nach Anspruch 1, wobei die Genotypinformation vom zweiten Elternteil phasierte Genotypinformation ist; welches ferner Folgendes umfasst:

- Kategorisieren der kontinuierlichen PVAF-Werte in eine zweite Kategorie, welche dem zweiten Elternteil entspricht, basierend auf der Genotypinformation vom ersten und zweiten Elternteil; und
- Subkategorisierung der kontinuierlichen PVAF-Werte in der ersten und in der zweiten Kategorie in Subkategorien.

**3.** Das Verfahren nach irgendeinem der vorigen Ansprüche, welches ferner das Reflektieren der erhaltenen PVAF-Werte gegen die Mittenachse vor der Segmentierung umfasst.

**4.** Das Verfahren nach Anspruch 1 oder 2, welches ferner das Reflektieren der erhaltenen PVAF-Werte umfasst, an einer Position, welche einem spezifischen phasierten parentalen Genotyp entspricht, gegen die Mittenachse vor der Segmentierung.

**5.** Das Verfahren nach irgendeinem der vorigen Ansprüche, welches ferner das Erhalten von DNA-Mengenwerten, wie logR-Werte oder ausgelesene Zählerstandswerte, und das Normalisieren der erwähnten DNA-Mengenwerte basierend auf den erwähnten segmentierten PVAF-Werten umfasst.

**6.** Das Verfahren nach irgendeinem der vorigen Ansprüche, wobei die kontinuierlichen PVAF-Werte bestimmt wurden, indem eine Probe verwendet wurde, welche eine geringe Menge von genetischem Material der erwähnten Testperson umfasst, wie zum Beispiel eine Probe, welche nur eine oder einige Zellen der erwähnten Testperson umfasst, oder eine Plasmaprobe erhalten von einer Mutter, die mit der erwähnten Testperson schwanger ist.

**7.** Das Verfahren nach irgendeinem der vorigen Ansprüche, wobei die genetische Anomalie, die durch die segmentierten PVAF-Werte kenntlich gemacht wurde, eine numerische oder strukturelle chromosomale Abweichung oder Mosaizismus umfasst.

**8.** Das Verfahren nach irgendeinem der vorigen Ansprüche, wobei die segmentierten PVAF-Werte den meiotischen oder mitotischen Ursprung einer genetischen Anomalie im genetischen Material der Testperson kenntlich machen.

9. Das Verfahren nach irgendeinem der vorigen Ansprüche, wobei die segmentierten PVAF-Werte den Haplotyp des genetischen Materials der Testperson kenntlich machen.

10. Das Verfahren nach irgendeinem der vorigen Ansprüche, wobei die segmentierten PVAF-Werte die Anzahl der Kopien eines Chromosoms oder einer Chromosomenregion im genetischen Material der Testperson und/oder eine homologe Rekombinationsstelle oder eine chromosomale Bruchstelle kenntlich machen.

11. Das Verfahren nach Anspruch 1 oder 2, wobei die Bereitstellung der segmentierten PVAF-Werte die Beurteilung der Länge von Segmenten von segmentierten PVAF-Werten in zumindest zwei Subkategorien der Kategorie eines Elternteils umfasst, um eine homologe Rekombinationsstelle oder eine chromosomale Bruchstelle kenntlich zu machen.

12. Das Verfahren nach Anspruch 2, wobei die Bereitstellung der segmentierten PVAF-Werte für eine Chromosomenregion Folgendes umfasst:

   - Bestimmen eines ersten Abstands zwischen a) einem segmentierten PVAF-Wert in einer ersten Subkategorie der Kategorie des ersten Elternteils in der erwähnten Chromosomenregion und b) einem segmentierten PVAF-Wert in einer zweiten Subkategorie der Kategorie des ersten Elternteils in der erwähnten Chromosomenregion;
   - Bestimmen eines zweiten Abstands zwischen a) einem segmentierten PVAF-Wert in einer ersten Subkategorie der Kategorie des zweiten Elternteils in der erwähnten Chromosomenregion und b) einem segmentierten PVAF-Wert in einer zweiten Subkategorie der Kategorie des zweiten Elternteils in der erwähnten Chromosomenregion; und
   - Vergleichen des ersten Abstands und des zweiten Abstands, um eine Anomalie der Anzahl der Kopien der erwähnten Chromosomenregion kenntlich zu machen.

13. Das Verfahren nach irgendeinem der vorigen Ansprüche, wobei die Kategorisierung der kontinuierlichen PVAF-Werte in eine Kategorie, welche dem ersten Elternteil entspricht, Folgendes umfasst:

   - Bestimmen von Loci, die informativ für den ersten Elternteil sind, indem die Genotypinformation vom ersten und zweiten Elternteil verwendet wird; und
   - Kategorisieren kontinuierlicher PVAF-Werte von genetischem Material der Testperson an den erwähnten Loci, die informativ für den ersten Elternteil in einer Kategorie, die dem ersten Elternteil entspricht, sind.

14. Das Verfahren nach Anspruch 1, wobei die Subkategorisierung der kontinuierlichen PVAF-Werte aus der Kategorie, welche dem ersten Elternteil entspricht, Folgendes umfasst:

   - Bestimmen von Loci mit einer spezifischen Genotypkombination vom ersten und zweiten Elternteil;
   - Subkategorisieren kontinuierlicher PVAF-Werte von genetischem Material der Testperson an den erwähnten Loci mit einer spezifischen Genotypkombination vom ersten und zweiten Elternteil.

15. Ein Produkt eines Computerprogramms, das, wenn es auf einem Prozessor installiert ist, in der Lage ist, das Verfahren nach irgendeinem der vorigen Ansprüche auszuführen.

16. Ein nicht-transitorisches maschinenlesbares Speichermedium, welches das Produkt des Computerprogramms aus Anspruch 15 speichert.

**Revendications**

1. Procédé implémenté par ordinateur pour l'analyse de matériel génétique d'un sujet, ledit procédé comprenant :

   - l'obtention de valeurs de fréquence d'allèles de variant polymorphe (PVAF) continues du matériel génétique du sujet ;
   - l'obtention d'informations de génotype en phase d'un premier parent et d'informations de génotype en phase ou non en phase d'un second parent ;
   - la catégorisation des valeurs PVAF continues dans une première catégorie correspondant au premier parent d'après les informations de génotype du premier et du second parent ;
   - la sous-catégorisation des valeurs PVAF continues à partir de la première catégorie correspondant au premier parent en sous-catégories ;
   - la segmentation desdites valeurs PVAF sous-catégorisées ; et
   - la fourniture des valeurs PVAF segmentées pour indiquer une anomalie génétique dans le matériel génétique du sujet et/ou un héritage du matériel génétique du sujet.

2. Procédé selon la revendication 1, dans lequel les informations de génotype du second parent sont des informations de génotype en phase ; comprenant en outre

- la catégorisation des valeurs PVAF continues en une seconde catégorie correspondant au second parent d'après les informations de génotype du premier et du second parent ; et

- la sous-catégorisation des valeurs PVAF continues dans les première et seconde catégories en sous-catégories.

3. Procédé selon l'une quelconque des revendications précédentes, comprenant en outre la réflexion de valeurs PVAF obtenues contre l'axe milieu avant segmentation.

4. Procédé selon la revendication 1 ou 2, comprenant en outre la réflexion de valeurs PVAF obtenues, à une position correspondant à un génotype parental en phase spécifique, contre l'axe milieu avant segmentation.

5. Procédé selon l'une quelconque des revendications précédentes, comprenant en outre l'obtention de valeurs de quantité d'ADN, telles que des valeurs logR ou des valeurs de comptage de lecture, et la normalisation desdites valeurs de quantité d'ADN d'après lesdites valeurs PVAF segmentées.

6. Procédé selon l'une quelconque des revendications précédentes, dans lequel les valeurs PVAF continues ont été déterminées à l'aide d'un échantillon comprenant une faible quantité de matériel génétique dudit sujet,

tel qu'un échantillon comprenant uniquement une ou quelques cellules dudit sujet, ou un échantillon de plasma obtenu auprès d'une mère enceinte dudit sujet.

7. Procédé selon l'une quelconque des revendications précédentes, dans lequel l'anomalie génétique indiquée par les valeurs PVAF segmentées comprend une anomalie chromosomique numérique ou structurelle ou un mosaïcisme.

8. Procédé selon l'une quelconque des revendications précédentes, dans lequel les valeurs PVAF segmentées indiquent l'origine méiotique ou mitotique d'une anomalie génétique dans le matériel génétique du sujet.

9. Procédé selon l'une quelconque des revendications précédentes, dans lequel les valeurs PVAF segmentées indiquent l'haplotype du matériel génétique du sujet.

10. Procédé selon l'une quelconque des revendications précédentes, dans lequel les valeurs PVAF segmentées indiquent le nombre de copies d'un chromosome ou d'une région chromosomique dans le matériel génétique du sujet et/ou un site de recombinaison homologue ou un point de rupture chromosomique.

11. Procédé selon la revendication 1 ou 2, dans lequel la fourniture des valeurs PVAF segmentées comprend l'évaluation de la longueur de segments de valeurs PVAF segmentées dans au moins deux sous-catégories de la catégorie d'un parent pour indiquer un site de recombinaison homologue ou un point de rupture chromosomique.

12. Procédé selon la revendication 2, dans lequel la fourniture des valeurs PVAF segmentées comprend pour une région chromosomique :

- la détermination d'une première distance entre a) une valeur PVAF segmentée dans une première sous-catégorie de la catégorie du premier parent au niveau de ladite région chromosomique et b) une valeur PVAF segmentée dans une seconde sous-catégorie de la catégorie du premier parent au niveau de ladite région chromosomique ;

- la détermination d'une seconde distance entre a) une valeur PVAF segmentée dans une première sous-catégorie de la catégorie du second parent au niveau de ladite région chromosomique et b) une valeur PVAF segmentée dans une seconde sous-catégorie de la catégorie du second parent au niveau de ladite région chromosomique ; et

- la comparaison de la première distance et de la seconde distance pour indiquer une anomalie de nombre de copies de ladite région chromosomique.

13. Procédé selon l'une quelconque des revendications précédentes, dans lequel la catégorisation des valeurs PVAF continues dans une catégorie correspondant au premier parent comprend :

- la détermination de loci qui sont informatifs du premier parent à l'aide des informations de génotype du premier et du second parent ; et

- la catégorisation de valeurs PVAF continues de matériel génétique du sujet au niveau desdits loci qui sont informatifs du premier parent dans une catégorie correspondant au premier parent.

14. Procédé selon la revendication 1, dans lequel la sous-catégorisation des valeurs PVAF continues à partir de la catégorie correspondant au premier parent comprend :

- la détermination de loci avec une combinaison de génotypes spécifique du premier et du second parent ;

- la sous-catégorisation de valeurs PVAF continues de matériel génétique du sujet au niveau desdits loci avec une combinaison de génotypes spécifique du premier et du second parent.

**15.** Produit de programme d'ordinateur qui est capable, lorsqu'il est exécuté sur un moteur de traitement, de réaliser le procédé de l'une quelconque des revendications précédentes.

**16.** Support de stockage lisible par machine non transitoire stockant le produit de programme d'ordinateur de la revendication 15.

EP 3 039 597 B1

Figure 1

Figure 2

Figure 3

Figure 4

Figure 5

Figure 6

Figure 7

Figure 8a

Figure 8b

Figure 9a

Nullisomy of chromosome 5 (E11-BI353-PGD008C1)

EP 3 039 597 B1

43

Figure 9b

Figure 9c

A normal disomic chromosome 9 (E05-BI632-PGD018)

Figure 9d

Trisomy of chromosome 7 (E05-BI131-PGD005)

Figure 10a

Trisomy of chromosome 1 (E11-BI091-PGD004C1)

Figure 10b

MI trisomy of chromosome 22 (E09-BI361-PGD008C1)

Figure 11a

Figure 11b

Figure 11c

Figure 11d

Maternal uniparental isodisomic chromosome 10

Paternal mitotic trisomy of chromosome 10

Figure 11e

Maternal meiotic trisomy of chromosome 22

Figure 12a

Genome-wide profile of a multi-cell DNA sample

Figure 12b

Chromosome 17 with mosaic paternal UPD ($d_{Pat} < d_{Mat}$)

Position (Mb)

Figure 13

Figure 13 continued

Figure 13 continued

Figure 14a

Figure 14b

Haplotyping of human exome-sequencing data

Exome sequencing data

SNP-array data

Figure 15a

Figure 15b

Chromosome X   (PVAF-profiles)

Figure 16a

Figure 16b

Figure 16b continued

Figure 17

Figure 17 continued

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• EP 1951897 A


**Non-patent literature cited in the description**

• **WANG et al.** PennCNV: an integrated hidden Markov model designed for high-resolution copy number variation detection in whole-genome SNP genotyping data. *Genome research,* 2007, vol. 17, 1665

• **VATTATHIL et al.** Haplotype-based profiling of subtle allelic imbalance with SNP arrays. *Genome research,* 2013, vol. 23, 152

• **SCHEET et al.** A fast and flexible statistical model for large-scale population genotype data: applications to inferring missing genotypes and haplotypic phase. *American journal of human genetics,* 2006, vol. 78, 629

• **NIK-ZAINAL et al.** The life history of 21 breast cancers. *Cell,* 2012, vol. 149, 5

• A map of human genome variation from population-scale sequencing. *Nature,* 2010, vol. 467, 1061

• **HOWIE et al.** Fast and accurate genotype imputation in genome-wide association studies through pre-phasing. *Nature Genetics,* 2012, vol. 44, 8

• **NAVIN et al.** Tumor evolution inferred by single-cell sequencing. *Nature,* 2011, vol. 472, 90

• **HANDYSIDE et al.** Karyomapping: a universal method for genome wide analysis of genetic disease based on mapping crossovers between parental haplotypes. *J Med Genet,* 2010, vol. 47, 10

• **VOET et al.** Single-cell paired-end genome sequencing reveals structural variation per cell cycle. *Nucleic Acids Res,* 2013, vol. 41, 12

• **VOET et al.** Breakage-fusion-bridge cycles leading to inv dup del occur in human cleavage stage embryos. *Human mutation,* 2011, vol. 32, 783